# EUROPEAN PATENT APPLICATION

(11) **EP 4 365 295 A1**
(43) Date of publication of application: **08.05.2024**
(21) Application number: 22832556.9
(22) Date of filing: 31.03.2022
(51) Int. Cl.: C12N 15/62, A61K 35/15, A61K 35/17, A61P 35/00, C07K 14/715, C07K 19/00, C12N 5/0783, C12N 5/0786, C12N 5/10, C12N 15/12, C12N 15/63

(54) **CHIMERIC CYTOKINE RECEPTOR**

(30) Priority: 28.06.2021 JP 2021106789
(71) Applicant: Aichi Prefecture, Nagoya-shi Aichi 460-0001 (JP)
(72) Inventor: KAGOYA, Yuki, Nagoya-shi, Aichi 464-8681 (JP); YOSHIKAWA, Toshiaki, Nagoya-shi, Aichi 464-8681 (JP)
(74) Representative: Mewburn Ellis LLP
(86) International application number: PCT/JP2022/016453
(87) International publication number: WO 2023/276395

(57) **Abstract**

To achieve both of improvement of therapeutic efficacy and reduction of side effects in adoptive immunotherapy, the present invention provides a novel technique for enhancing functions such as proliferation ability and imparting an activity of capturing cytokines which may cause side effects in immune cells for use in the adoptive immunotherapy. Provided is a chimeric cytokine receptor comprising a ligand-binding region at the N-terminal side and a T-cell activating region at the C-terminal side, wherein: said ligand-binding region consists of a cytokine-binding region of a cytokine receptor, said T-cell activating region comprises the transmembrane domain and the intracellular domain of an IL-7 (Interleukin-7) receptor α chain, and said transmembrane domain has an insertion of any one selected from the group consisting of (a) the amino acid sequence of SEQ ID NO: 2 between position 243 and position 244 in the amino acid sequence of SEQ ID NO: 1, (b) the amino acid sequence of SEQ ID NO: 3 between position 241 and position 242 in the amino acid sequence of SEQ ID NO: 1, (c) the amino acid sequence of SEQ ID NO: 4 between position 244 and position 245 in the amino acid sequence of SEQ ID NO: 1, (d) the amino acid sequence of SEQ ID NO: 5 between position 244 and position 245 in the amino acid sequence of SEQ ID NO: 1, and (e) the amino acid sequence of SEQ ID NO: 6 between position 246 and position 247 in the amino acid sequence of SEQ ID NO: 1.

## Description

### Technical Field

The present invention relates to a chimeric cytokine receptor and a method for producing a chimeric antigen receptor (CAR)-transduced cell having a long-lasing cytotoxic activity.

### Background Art

Adoptive immunotherapy is a therapeutic method that comprises collecting immune cells from a donor, such as a patient himself/herself, culturing, stimulating, manipulating, and/or growing the cells *in vitro,* and then introducing the cells into the patient. Examples of the adoptive immunotherapy include tumor infiltrating lymphocyte therapy, T-cell receptor-T (TCR-T) cell therapy, and chimeric antigen receptor-T (CAR-T) cell therapy for a patient with cancer.

The CAR-T cell therapy among them is a method which comprises introducing into T cells a chimeric antigen receptor (CAR) in which a single-chain antibody recognizing a cell surface antigen of cancer is combined with a transmembrane domain and a signaling domain of a co-stimulatory molecule involved in T cell activation. The CAR-T cell therapy is designed to kill tumor cells by introducing a CAR gene encoding a CAR into T cells which is collected from and then returned to a patient.

Currently, in the CAR-T cell therapy, a therapeutic method targeting the CD19 molecule in B-cell leukemia or lymphoma is approved in the United States, Europe, and Japan. With an extremely high response rate, the therapy has been reported to achieve remission greater than 80% even in refractory cases. In addition, CAR-T cell therapy targeting other antigen and cancer types are being developed, and the scale of the market is expected to expand in the future.

The adoptive immunotherapy is expected as a treatment method which may achieve cure of recurrent and refractory tumors but has not yet exhibited sustained therapeutic efficacy on many cancers except for some cancer types. The reasons why sustained therapeutic efficacy is not obtained in the CAR-T cell therapy include attenuation of the function such as proliferative ability in therapeutic cells.

Furthermore, side effects such as cytokine release syndrome and neurotoxicity syndrome have also become problems in the adoptive immunotherapy. In cytokine release syndrome associated with CAR-T cell therapy, cytokines such as IL-6 are released from e.g., macrophages activated by e.g., GM-CSF secreted from CAR-T cells, resulting in a decrease in blood pressure and multiple organ dysfunction (Non Patent Literatures 1 and 2). In addition, cytokines such as IL-1β are released from e.g., macrophages activated by CAR-T cells in the neurotoxicity syndrome, resulting in e.g., aphasia, mental abnormality, convulsion, and cerebral edema (Non Patent Literatures 3 and 4).

Accordingly, there are two issues in the adoptive immunotherapy as represented by the CAR-T cell therapy: improvement of therapeutic efficacy and reduction of side effects. However, in the adoptive immunotherapy, it is known that the improvement of therapeutic efficacy correlates with the event frequency and grade of the side effects. Therefore, it is difficult to achieve both improvement of therapeutic efficacy and reduction of side effects at the same time since it is thought that the side effects increase if the therapeutic efficacy is increased, and conversely, the therapeutic efficacy decreases if the side effects are reduced.

Therefore, there is a need for a novel technique for achieving both of improvement of therapeutic efficacy and reduction of side effects in the adoptive immunotherapy.

### Citation List

### Non Patent Literature

Non Patent Literature 1: Maude S.L., et al., N Engl J Med, 2018, 378(5):439-448.
Non Patent Literature 2: Sachdeva M., et al., J Biol Chem., 2019, 294(14):5430-5437.
Non Patent Literature 3: Giavridis T, et al., Nat Med., 2018, 24(6):731-738.
Non Patent Literature 4: Norelli M., et al., Nat Med, 2018, 24(6):739-748.

### Summary of Invention

### Technical Problem

An object of the present invention is to provide a novel technique for enhancing functions such as proliferative ability of immune cells for use in the adoptive immunotherapy and imparting an activity for capturing cytokines which can cause side effects, to immune cells in order to achieving both of improvement of therapeutic efficacy and reduction of side effects in the adoptive immunotherapy.

### Solution to Problem

To solve the above problems, the present inventors have produced a chimeric cytokine receptor based on a plurality of cytokine receptors as a new artificial receptor that enables both enhancement of functions such as proliferative ability and cytokine capture in a CAR-T cell. This chimeric cytokine receptor is composed of a cytokine-binding region for cytokine capture at the N-terminal side and a T-cell activating region based on a constitutively active IL-7 (Interleukin-7) receptor α chain at the C-terminal side. The present inventors have found that a CAR-T cell into which the same chimeric cytokine receptor is introduced together with a chimeric antigen receptor (CAR) has enhanced functions such as proliferative ability and cytotoxic activity, and that the same CAR-T cell acquires an activity of capturing the extracellular cytokine. The present inventors have also found that the CAR-T cell into which the chimeric cytokine receptor of the present invention is introduced together with the CAR exhibits a long-term survival ability and long-lasting anti-tumor effects *in vivo.*

On the other hand, when another chimeric cytokine receptor was produced based on a constitutively active CD28 receptor which is known to contribute to enhancement of functions of T cells such as proliferative ability and cytotoxic activity, similar to the constitutively active IL-7 receptor α chain, the above effects were not obtained. Accordingly, it was found to be important that the T-cell activating region of the chimeric cytokine receptor is based on the constitutively active IL-7 receptor α chain rather than the constitutively active CD28 receptor, for obtaining the above effects.

The present invention is based on the above-described research results and provides the following.
(1) A chimeric cytokine receptor comprising a ligand-binding region at the N-terminal side and a T-cell activating region at the C-terminal side, wherein:
   said ligand-binding region consists of a cytokine-binding region of a cytokine receptor,
   said T-cell activating region comprises the transmembrane domain and the intracellular domain of an IL-7 (Interleukin-7) receptor α chain, and
   said transmembrane domain has an insertion of any one selected from the group consisting of
      (a) the amino acid sequence of SEQ ID NO: 2 between position 243 and position 244 in the amino acid sequence of SEQ ID NO: 1,
      (b) the amino acid sequence of SEQ ID NO: 3 between position 241 and position 242 in the amino acid sequence of SEQ ID NO: 1,
      (c) the amino acid sequence of SEQ ID NO: 4 between position 244 and position 245 in the amino acid sequence of SEQ ID NO: 1,
      (d) the amino acid sequence of SEQ ID NO: 5 between position 244 and position 245 in the amino acid sequence of SEQ ID NO: 1, and
      (e) the amino acid sequence of SEQ ID NO: 6 between position 246 and position 247 in the amino acid sequence of SEQ ID NO: 1.
(2) The chimeric cytokine receptor of (1), wherein said cytokine receptor is selected from the group consisting of an IL-6 (Interleukin-6) receptor, an IL-1 (Interleukin-1) receptor type 2, a Granulocyte macrophage colony-stimulating factor (GM-CSF) receptor α chain, and a GM-CSF receptor β chain.
(3) The chimeric cytokine receptor of (2), wherein said cytokine receptor is an IL-6 receptor and further comprises the ligand-binding region of gp130 (Glycoprotein 130) at the N-terminal side thereof.
(4) The chimeric cytokine receptor of any one of (1) to (3), having a mutation which reduces the activity of one or more motifs selected from the group consisting of a JAK-binding motif, a STAT3 association motif, and a STAT5/PI3K association motif which are contained in said intracellular domain.
(5) The chimeric cytokine receptor of (4), wherein said mutation is Y449F, M452L, or Y456F in the amino acid sequence of SEQ ID NO: 1.
(6) A nucleic acid encoding the chimeric cytokine receptor of any one of (1) to (5).
(7) A gene expression vector comprising the nucleic acid of (6) in a state which allows for the expression thereof.
(8) A host cell comprising the gene expression vector of (7).
(9) The host cell of (8), further comprising a chimeric antigen receptor (CAR) expression vector which comprises a base sequence encoding a CAR in a state which allows for the expression thereof.
(10) The host cell of (8) or (9), further comprising an IL-1 receptor type 2 expression vector which comprises a base sequence encoding a full-length IL-1 receptor type 2 in a state which allows for the expression thereof.
(11) The host cell of any one of (8) to (10) which is an immune cell.
(12) The host cell of (11), wherein said immune cell is a T cell, an NK cell, or a macrophage.
(13) A cell preparation comprising the host cell of any one of (8) to (12).
(14) A method of producing a chimeric antigen receptor (CAR)-transduced cell having a long-lasing cytotoxic activity, comprising: an isolating step of isolating peripheral blood mononuclear cells from the peripheral blood of a subject, and an introducing step of introducing the gene expression vector of (7) and a CAR-expression vector to the peripheral blood mononuclear cells isolated in said isolating step, wherein said CAR-expression vector comprises a base sequence encoding a chimeric antigen receptor (CAR) in a state which allows for the expression thereof.

The present specification encompasses the contents disclosed in Japanese Patent Application No. 2021-106789, which is the basis of the priority of the present application.

### Advantageous Effects of Invention

The chimeric cytokine receptor of the present invention can be introduced into an immune cell for use in the adoptive immunotherapy to enhance its functions such as proliferative ability. In addition, the immune cell into which the chimeric cytokine receptor of the present invention is introduced can capture extracellular cytokines.

### Brief Description of Drawings

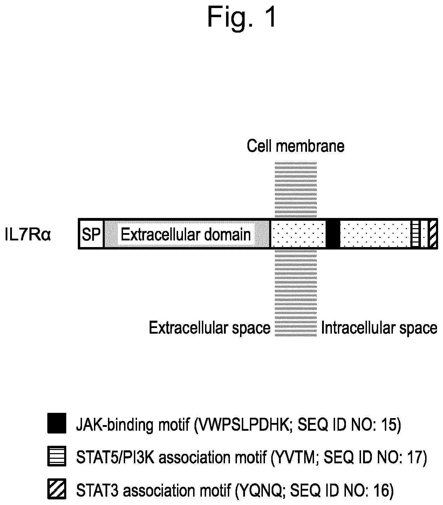

[Figure 1] Figure 1 shows the structure of an IL-7 receptor α chain. In the figure, SP represents a signal peptide.

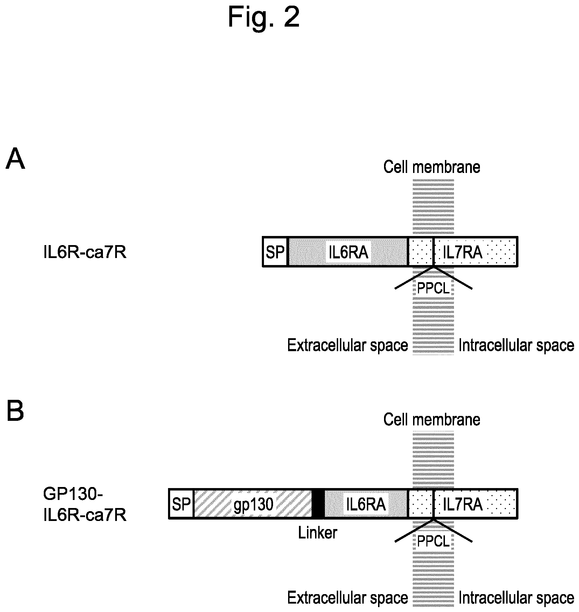

[Figure 2] Figure 2 shows the structures of chimeric cytokine receptors IL6R-ca7R and GP130-IL6R-ca7R. Figure 2A shows the structure of IL6R-ca7R. Figure 2B shows the structure of GP130-IL6R-ca7R. In the figure, SP represents a signal peptide, and PPCL represents four-amino acid residues Pro-Pro-Cys-Leu (SEQ ID NO: 2), which is a constitutively active insertion mutation.

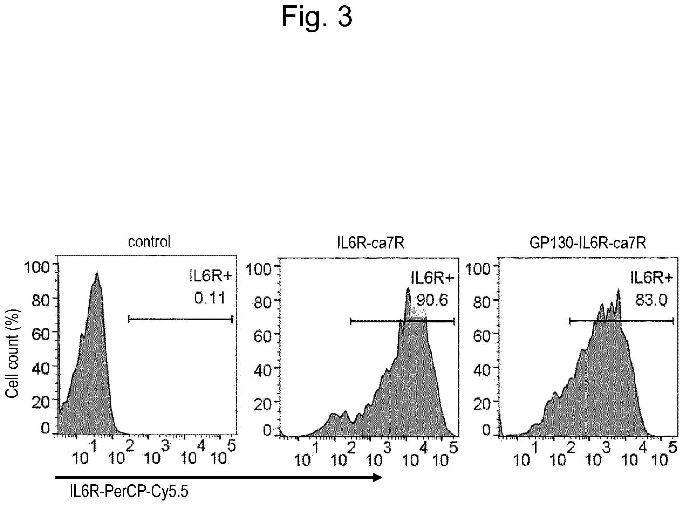

[Figure 3] Figure 3 shows a flow cytometry plot in which the cell surface expression of each receptor of IL6RA, IL6R-ca7R and/or GP130-IL6R-ca7R has been analyzed in CAR-T cells five days after retroviral transduction of an IL6R-ca7R gene or a GP130-IL6R-ca7R gene and in control cells. In the figure, the value shown as "IL6RA+" indicates the proportion (%) of the cells determined as positive based on the anti-IL6RA antibody.

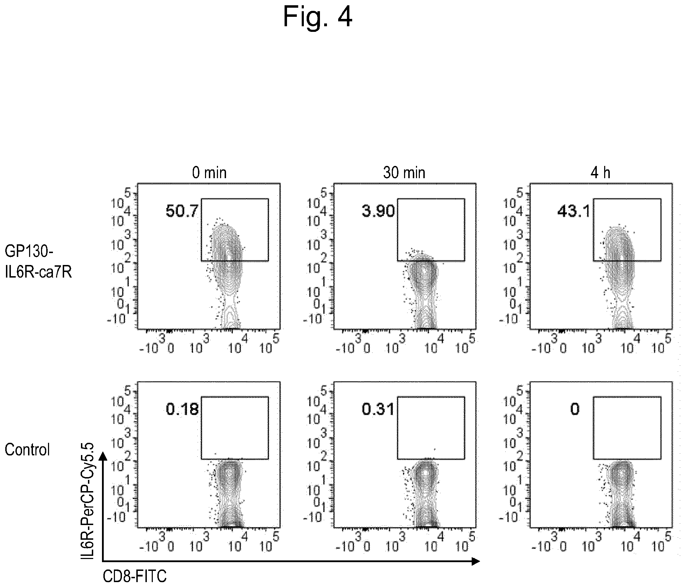

[Figure 4] Figure 4 shows results of flow cytometry analysis on receptor internalization kinetics at each time point after administration of recombinant IL-6 to GP130-IL6R-ca7R-transduced CAR-T cells or control cells.

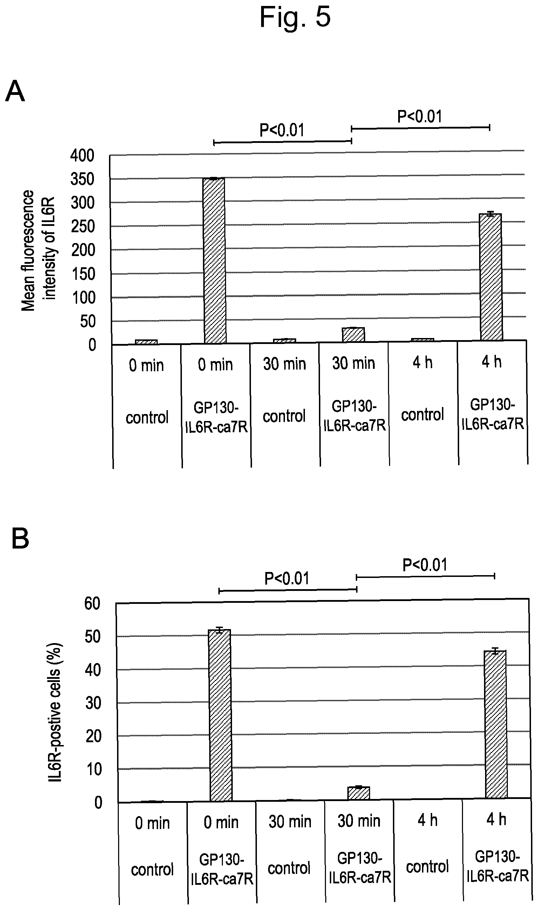

[Figure 5] Figure 5 shows quantification results of flow cytometry. Figure 5A shows fluorescence intensity detected by the anti-IL6RA antibody at each time point after the administration of IL-6 to GP130-IL6R-ca7R-transduced CAR-T cells or control cells. Figure 5B shows the proportion of positive cells detected by the anti-IL6RA antibody. The results show mean values of experiments for n = 4, and error bars indicate standard deviation. P values shown in the figure indicate statistical significance by one-way ANOVA.

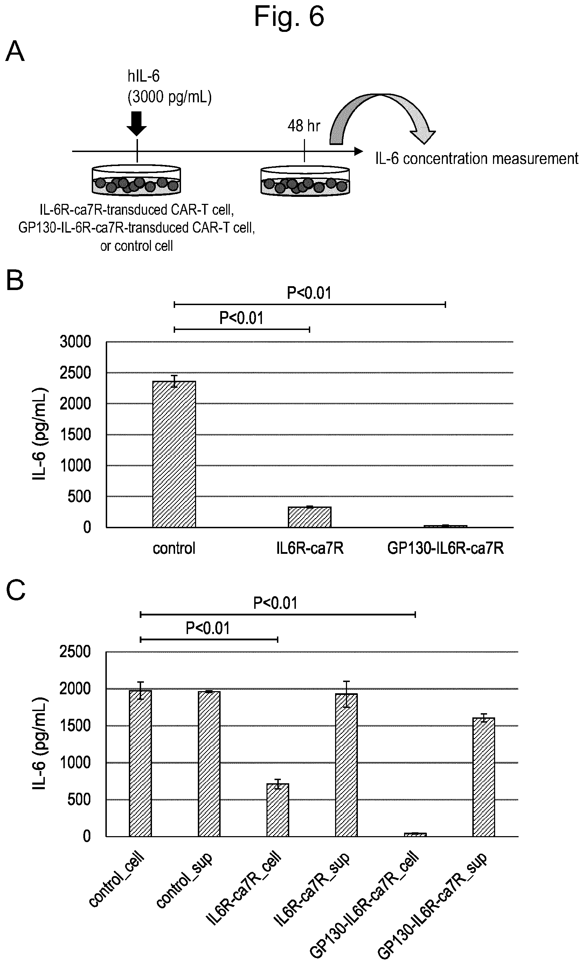

[Figure 6] Figure 6 shows measurement results of cytokine (IL-6) capturing ability of each CAR-T cell. Figure 6A shows the experimental procedure. Figure 6B shows the results of measuring IL-6 concentration in the supernatant of each CAR-T cell to which IL-6 was added. Figure 6C shows results of the measurements carried out in the same manner as in Figure 6B, in "control_cell," "IL6R-ca7R_cell," and "GP130-IL6R-ca7R_cell". In addition, the results of measuring the IL-6 concentration in the supernatant after addition of IL-6 to the culture supernatant isolated from each CAR-T cell are shown in "control sup," "IL6R-ca7R_sup," and "GP130-IL6R-ca7R_sup." The results show mean values of experiments for n = 3, and error bars indicate standard deviation. P values shown in the figure indicate statistical significance by one-way ANOVA.

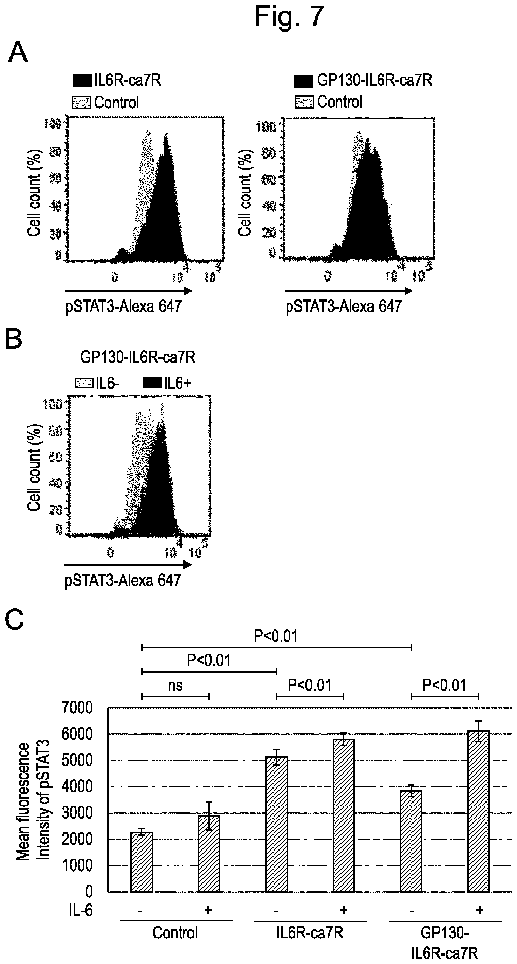

[Figure 7] Figure 7 shows results of evaluating phosphorylated STAT3 (pSTAT3) in each CAR-T cell. Figure 7A shows results of flow cytometry based on pSTAT3 in each CAR-T cell. Figure 7B shows results of flow cytometry based on pSTAT3 in the presence (IL6+) or absence (IL6-) of IL-6 in each CAR-T cell. Figure 7C shows quantification results of pSTAT3. The results show mean values of experiments for n = 4, and error bars indicate standard deviation. P values shown in the figure indicate statistical significance by one-way ANOVA.

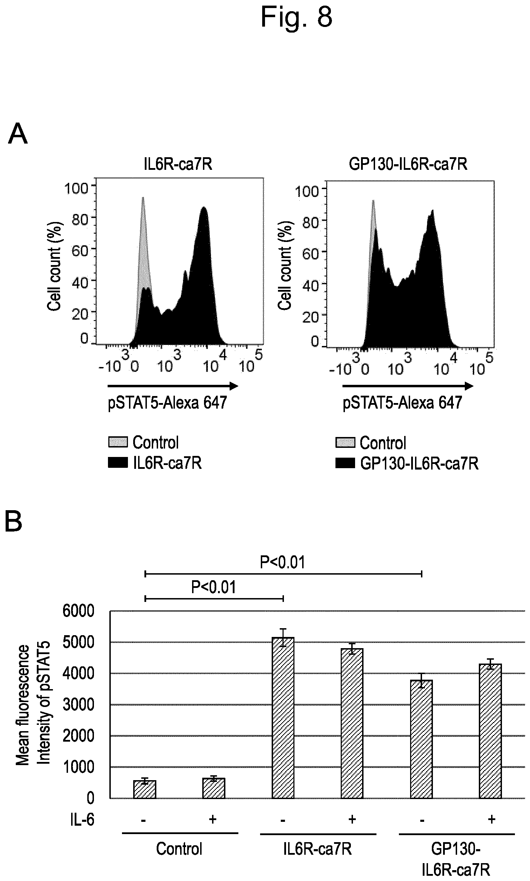

[Figure 8] Figure 8 shows results of evaluating phosphorylated STATS (pSTAT5) in each CAR-T cell. Figure 8A shows results of flow cytometry based on pSTAT5 in the absence of IL-6 in each CAR-T cell. Figure 8B shows quantification results of pSTAT5 in the presence or absence of IL-6. The results show mean values of experiments for n = 4, and error bars indicate standard deviation. P values shown in the figure indicate statistical significance by one-way ANOVA.

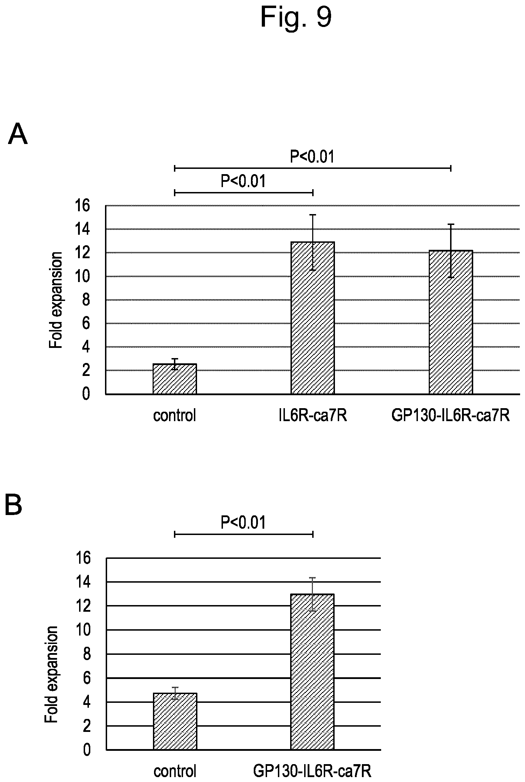

[Figure 9] Figure 9 shows cell proliferation rate of each CAR-T cell. CAR-T cells targeting CD19 were each cultured with CD19-positive tumor cell line NALM6, and the fold change of the cell number of CAR-T cells after seven days relative to that of the start of co-culture. is shown Figure 9A shows the results of FMC63-28z CAR-transduced CAR-T cells. The results show mean values of experiments for n = 4, and error bars indicate standard deviation. P values shown in the figure indicate statistical significance by one-way ANOVA. Figure 9B shows results of FMC63-BBz CAR-transduced CAR-T cell. The results show mean values of experiments for n = 4, and error bars each indicate standard deviation. P values shown in the figure indicate statistical significance by Student's t-test.

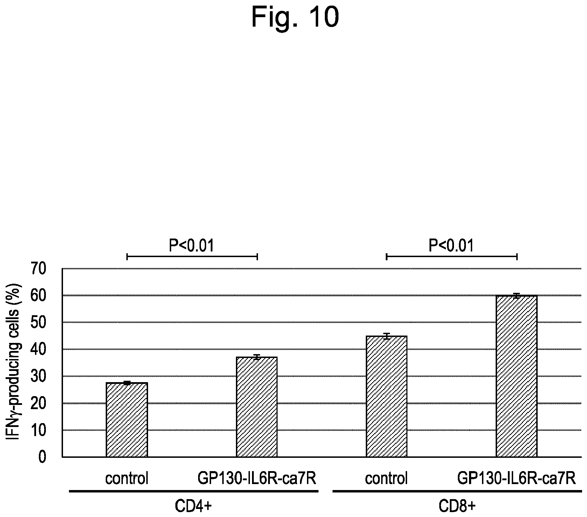

[Figure 10] Figure 10 shows quantification results of the proportion of IFN-γ-producing cells in the CD4-positive T cell fraction and the CD8-positive T cell fraction after each CAR-T cell was co-cultured with the CD19-positive tumor cell line, NALM6. The results show mean values of experiments for n = 4, and error bars indicate standard deviation. P values shown in the figure indicate statistical significance by Student's t-test.

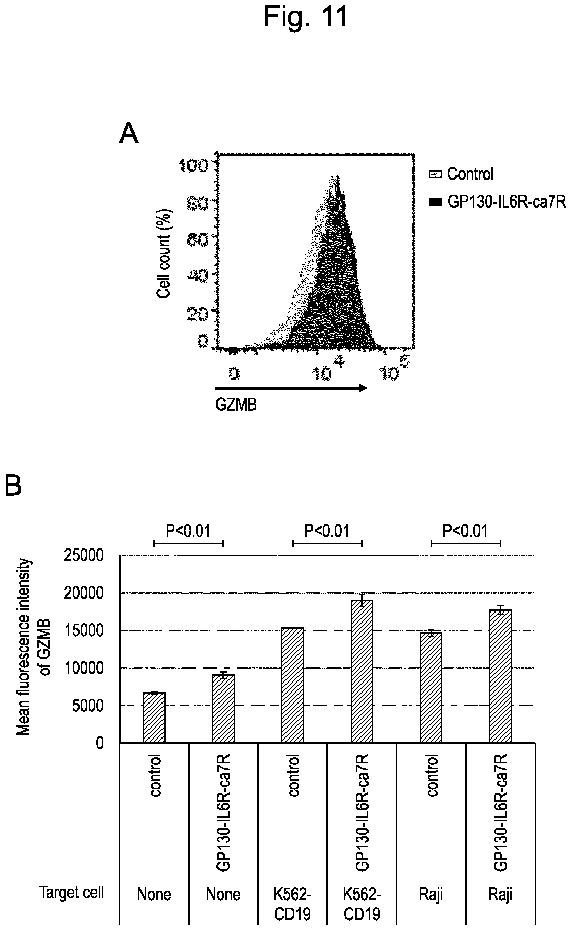

[Figure 11] Figure 11 shows results of analyzing the Granzyme B (GZMB) production amount after each CAR-T cell was co-cultured with the K562-CD19 cell line or Raji cell line. Figure 11A shows results of analyzing the Granzyme B production amount after co-culture with the K562-CD19 cell line. Figure 11B shows results of detecting the Granzyme B production amount in co-culture with different target cells. The results show mean values of experiments for n = 3, and error bars indicate standard deviation. P values shown in the figure indicate statistical significance by Student's t-test.

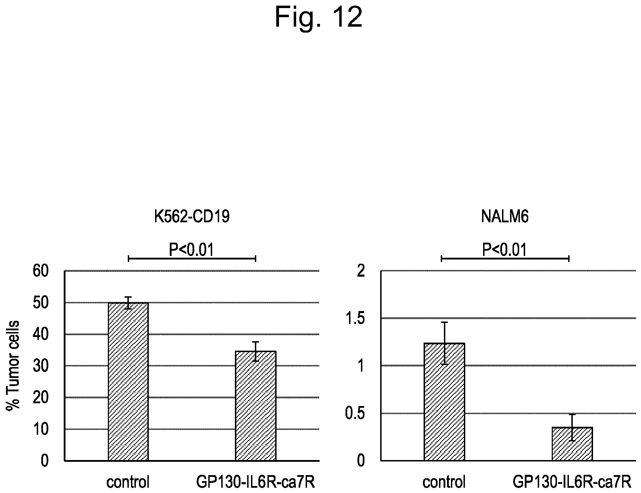

[Figure 12] Figure 12 shows survival rate of tumor cells (K562-CD19 cell line or NALM6 cell line) co-cultured with each CAR-T cell. The results show mean values of experiments for n = 3, and error bars indicate standard deviation. P values shown in the figure indicate statistical significance by Student's t-test.

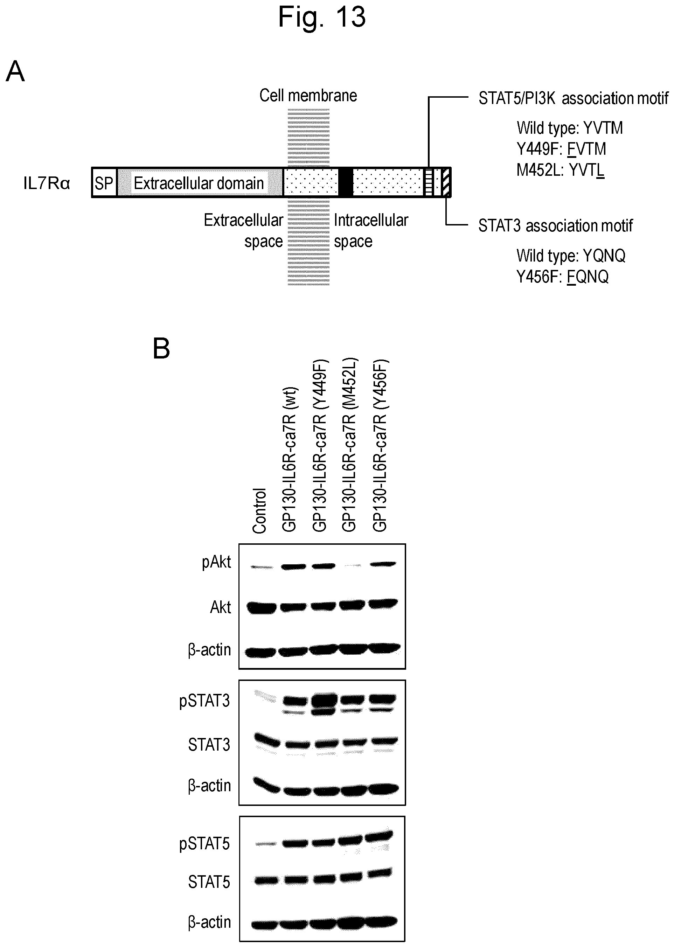

[Figure 13] Figure 13 shows the effects of a suppressive mutation Y449F, M452L, or Y456F in the chimeric cytokine receptor GP130-IL6R-ca7R. Figure 13A shows positions of Y449F, M452L, and Y456F in the IL-7 receptor α chain. Figure 13B shows results of Western blot analysis for STAT3, STAT5, and Akt phosphorylation states in CAR-T cells co-transduced with chimeric cytokine receptor GP130-IL6R-ca7R having each mutation.

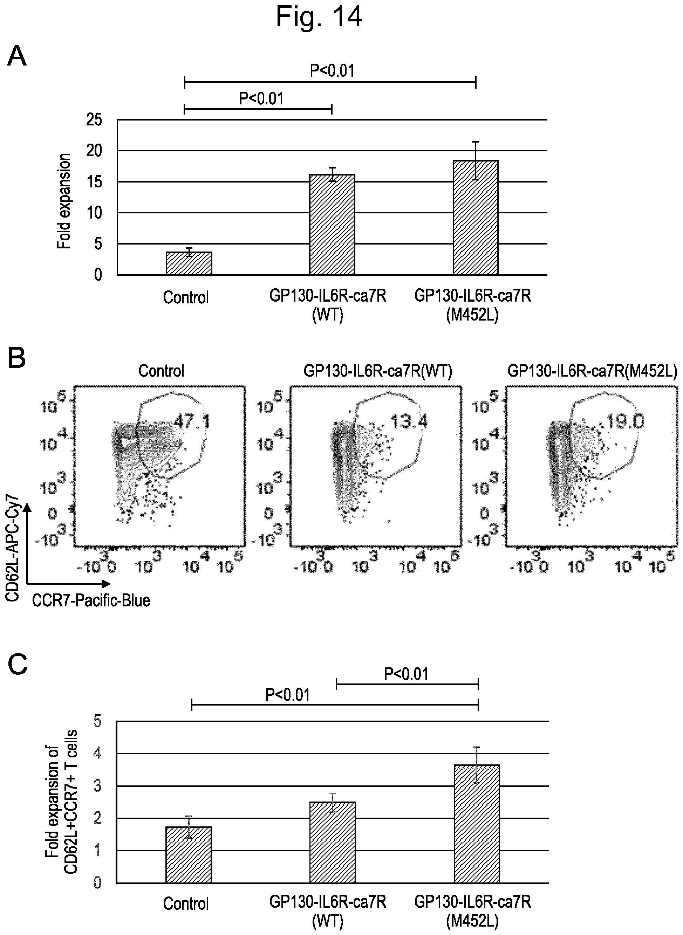

[Figure 14] Figure 14 shows results of functional analysis of CAR-T cells co-transduced with chimeric cytokine receptor GP130-IL6R-ca7R (M452L). Figure 14A shows the fold change in the number of each CAR-T cell after seven days of co-culture with CD19-positive tumor cell line NALM6. P values shown in the figure indicate statistical significance by one-way ANOVA. Figure 14B shows results of flow cytometry analysis of the memory phenotype of each CAR-T cell after seven days of co-culture with NALM6. Figure 14C shows the fold change in the number of the CAR-T cells having immature memory phenotypes (CD62L-positive and CCR7-positive). P values indicate statistical significance by one-way ANOVA.

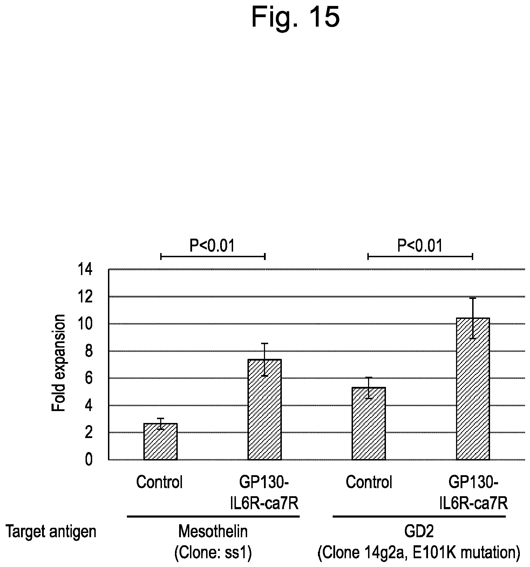

[Figure 15] Figure 15 shows cell proliferation rate of each CAR-T cell. CAR-T cells targeting mesothelin or GD2 were each cultured with the K562-mesothelin cell line or NALM6-GD2 cell line, and the fold change in the number of the CAR-T cells after seven days relative to the start of co-culture is shown. The results show mean values of experiments for n = 4, and error bars indicate standard deviation. P values shown in the figure indicate statistical significance by Student's t-test.

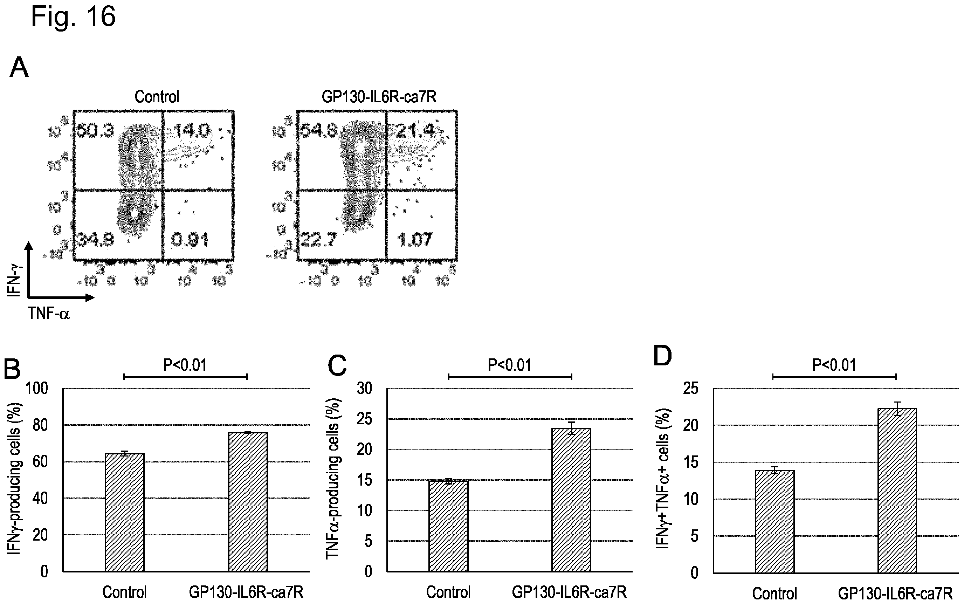

[Figure 16] Figure 16 shows results of flow cytometry analysis of CAR-T cells targeting mesothelin each of which was co-cultured with the K562-mesothelin cell line and stained with anti-IFN-γ and anti-TNF-α antibodies. Figure 16A shows the results of flow cytometry. Figure 16B shows the proportion of IFN-γ-producing cells. Figure 16C shows the proportion of TNF-α-producing cells. Figure 16D shows cells producing both IFN-γ and TNF-α. The results show mean values of experiments for n = 3, and error bars indicate standard deviation. P values shown in the figure indicate statistical significance by Student's t-test.

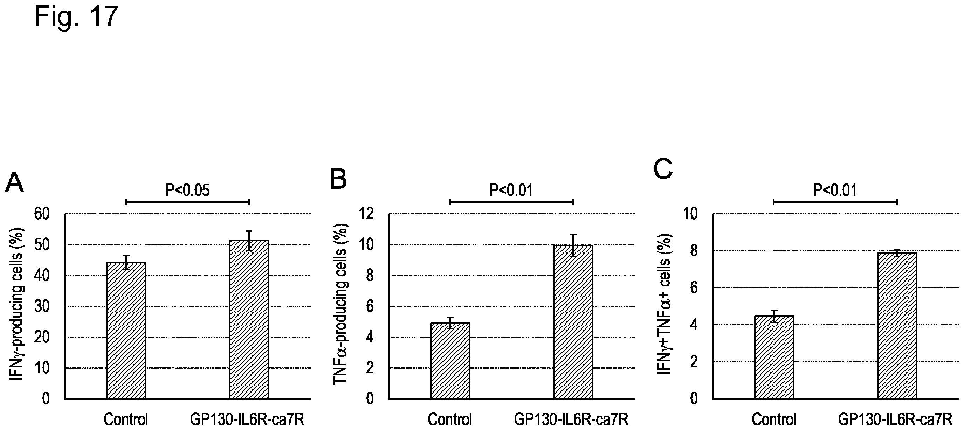

[Figure 17] Figure 17 shows results of flow cytometry analysis of CAR-T cells targeting GD2 each of which was co-cultured with the NALM6-GD2 cell line and stained with anti-IFN-γ and anti-TNF-α antibodies. Figure 17A shows the proportion of IFN-γ-producing cells. Figure 17B shows the proportion of TNF-α-producing cells. Figure 17C shows cell producing both IFN-γ and TNF-α. The results show mean values of experiments for n = 3, and error bars indicate standard deviation. P values shown in the figure indicate statistical significance by Student's t-test.

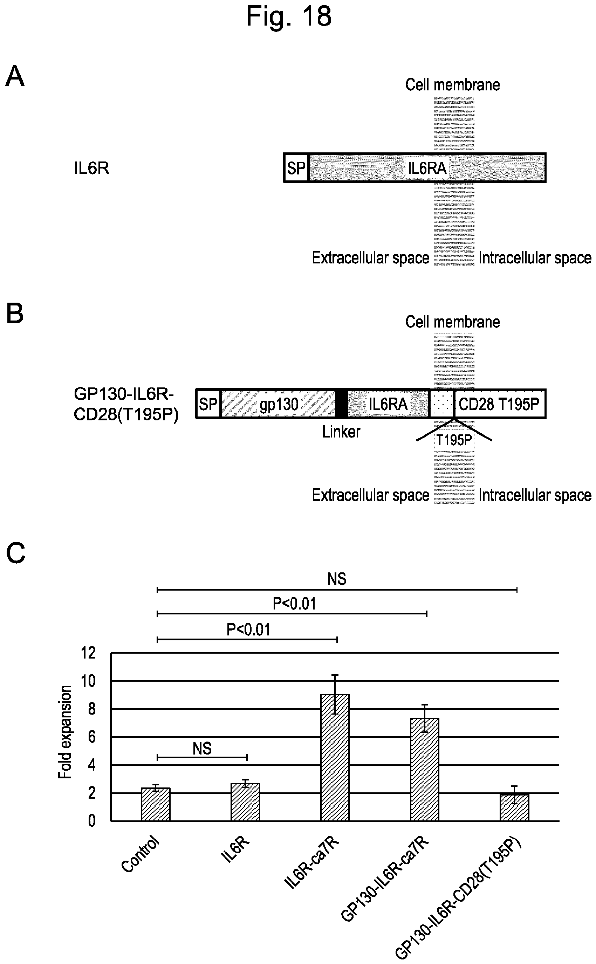

[Figure 18] Figure 18 shows the structure of chimeric cytokine receptor GP130-IL6R-CD28 (T195P). Figure 18A shows the structure of IL6 receptor α subunit (IL6RA). Figure 18B shows the structure of GP130-IL6R-CD28(T195P). In the figure, SP represents a signal peptide, T195P indicates a constitutively active mutation of CD28. Figure 18C shows cell proliferation rate of each CAR-T cell. CAR-T cells targeting CD19 were each cultured with CD19-positive tumor cell line NALM6, and the fold change in the number of the CAR-T cells after seven days relative to the start of co-culture is shown. The results show mean values of experiments for n = 4, and error bars indicate standard deviation. Statistical significance was tested by one-way ANOVA.

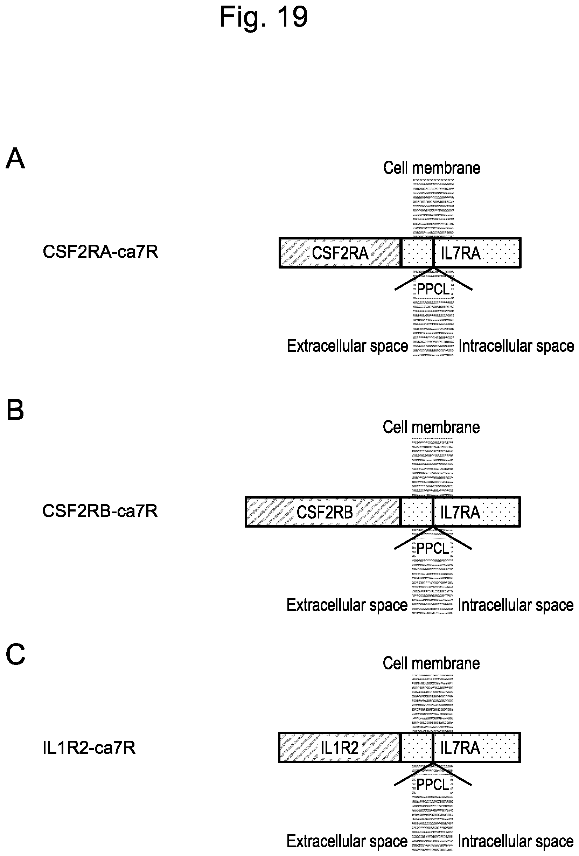

[Figure 19] Figure 19 shows the structures of chimeric cytokine receptors CSF2RA-ca7R, CSF2RB-ca7R, and IL1R2-ca7R. Figure 19A shows the structure of CSF2RA-ca7R. Figure 19B shows the structure of CSF2RB-ca7R. Figure 19C shows the structure of IL1R2-ca7R. In the figure, PPCL represents a constitutively active insertion mutation of four-amino acid residues Pro-Pro-Cys-Leu (SEQ ID NO: 2).

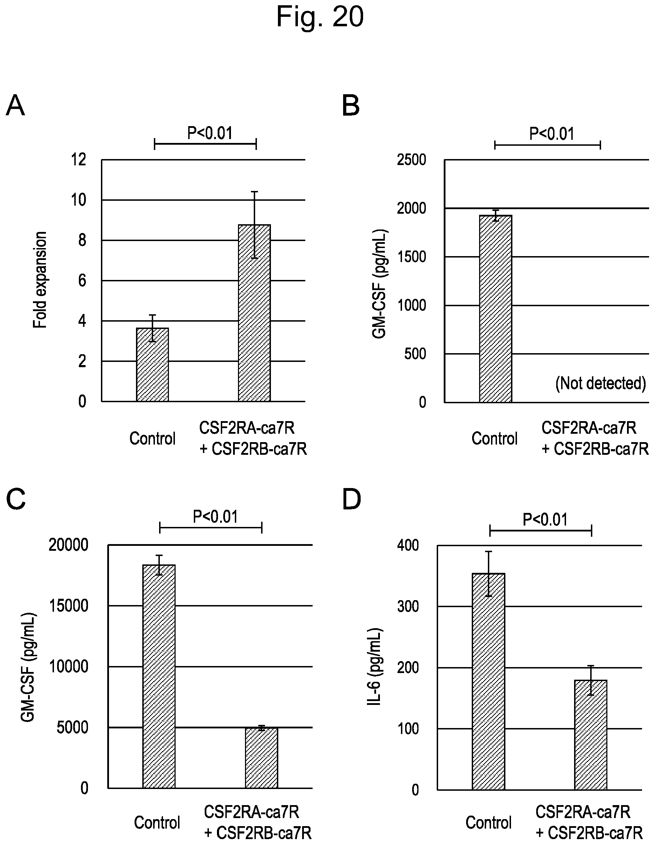

[Figure 20] Figure 20 shows results of functional analysis of CAR-T cells co-transduced with both CSF2RA-ca7R and CSF2RB-ca7R. Figure 20A shows the fold change in the number of CAR-T cells after seven days of co-culturing each CAR-T cell with CD19-positive tumor cell line NALM6. Figure 20B shows measurement results of GM-CSF capturing ability of each CAR-T cell. The results of adding GM-CSF to each CAR-T cell to measure GM-CSF concentration in the supernatant thereof are shown. Figures 20C and 20D show results of measuring GM-CSF concentration (Figure 20C) and IL-6 concentration (Figure 20D) in the culture liquid after co-culturing each CAR-T cell with NALM6 cells and then adding the monocytic leukemia cell line THP1. P values shown in the figure indicate statistical significance by Student's t-test.

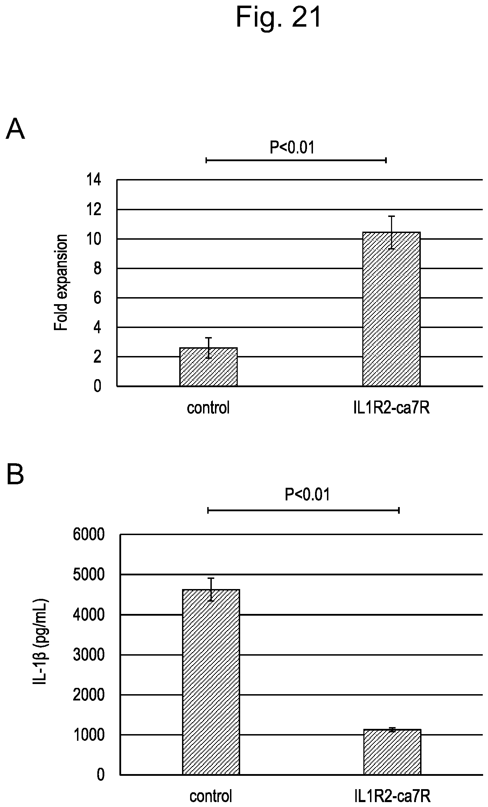

[Figure 21] Figure 21 shows measurement results of cell proliferation rate and cytokine (IL-1β) capturing ability of each CAR-T cell. Figure 21A shows the fold change in the number of CAR-T cells after seven days of culturing each of the CAR-T cells targeting CD19 with CD19-positive tumor cell line NALM6. The results show mean values of experiments for n = 4, and error bars indicate standard deviation. P values shown in the figure indicate statistical significance by Student's t-test. Figure 21B shows the results of adding IL-1β to each CAR-T cell to measure IL-1β concentration in the supernatant thereof. The results show mean values of experiments for n = 3, and error bars indicate standard deviation. P values shown in the figure indicate statistical significance by Student's t-test.

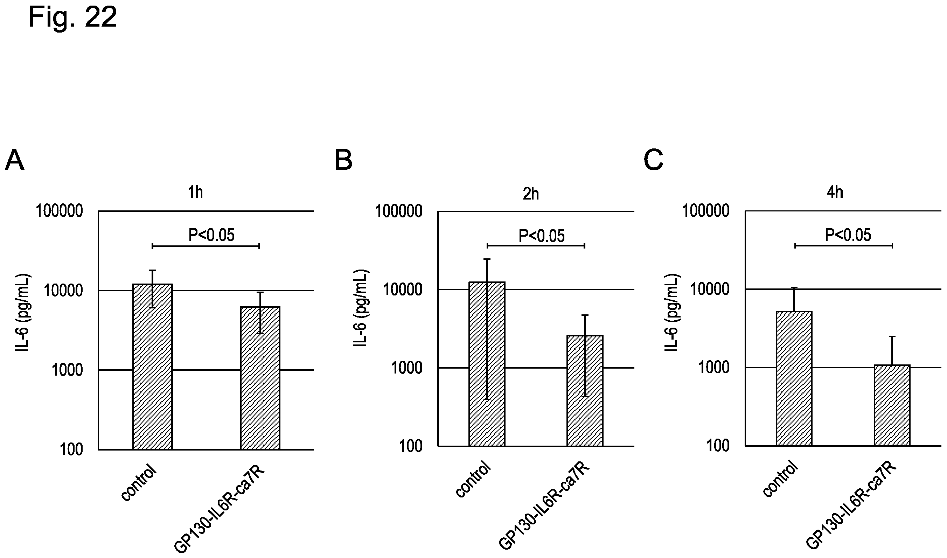

[Figure 22] Figure 22 shows results of measuring blood IL-6 concentrations one hour, two hours, and four hours after tail vein administration of human IL-6 to NSG mice treated with each CAR-T cell. P values shown in the figure indicate statistical significance by Student's t-test of logarithmic values of concentration. Figure 22A shows the blood IL-6 concentration after one hour. Figure 22B shows the blood IL-6 concentration after two hours. Figure 22C shows the blood IL-6 concentration after four hours.

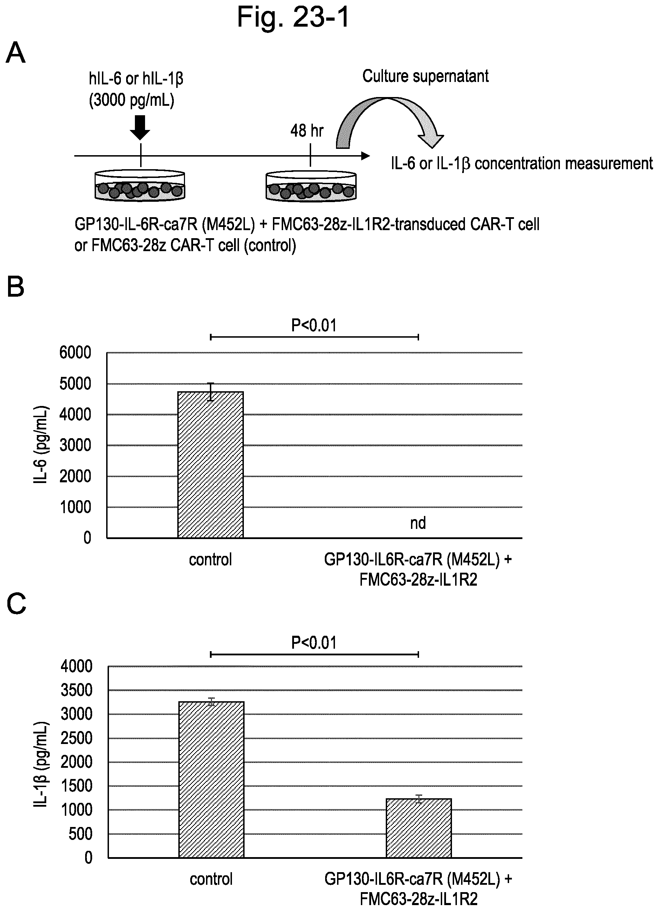

[Figure 23-1] Figure 23-1 shows measurement results of cytokine (IL-6 and IL-1β) capturing ability of CAR-T cells co-transduced with a chimeric cytokine receptor and full-length IL-1 receptor type 2. Figure 23-1A shows the experimental procedure. Figure 23-1B shows results of adding recombinant IL-6 to the CAR-T cells to measure IL-6 concentration in the supernatant thereof. "nd" indicates that IL-6 was not detected. Figure 23-1C shows results of adding recombinant IL-1β to the above CAR-T cells to measure IL-1β concentration in the supernatant thereof. The results show mean values of experiments for n = 3, and error bars indicate standard deviation. P values shown in the figure indicate statistical significance by Student's t-test.

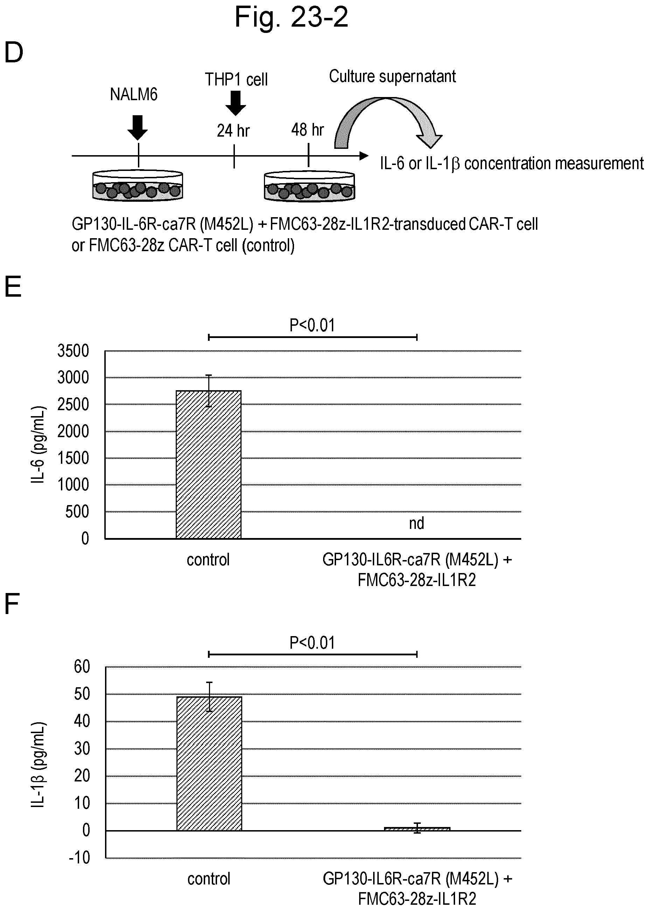

[Figure 23-2] Figure 23-2 is a continuation of Figure 23-1. Figure 23-2D shows the experimental procedure. Figure 23-2E shows results of adding monocytic cell line THP-1 to the above CAR-T cells cultured with CD19-positive tumor cell line NALM6 to measure the IL-6 concentration in the supernatant thereof. "nd" indicates that IL-6 was not detected. Figure 23-2F shows results of adding monocytic cell line THP-1 to the CAR-T cells to measure IL-1β concentration in the supernatant thereof. The results show mean values of experiments for n = 3, and error bars indicate standard deviation. P values shown in the figure indicate statistical significance by Student's t-test.

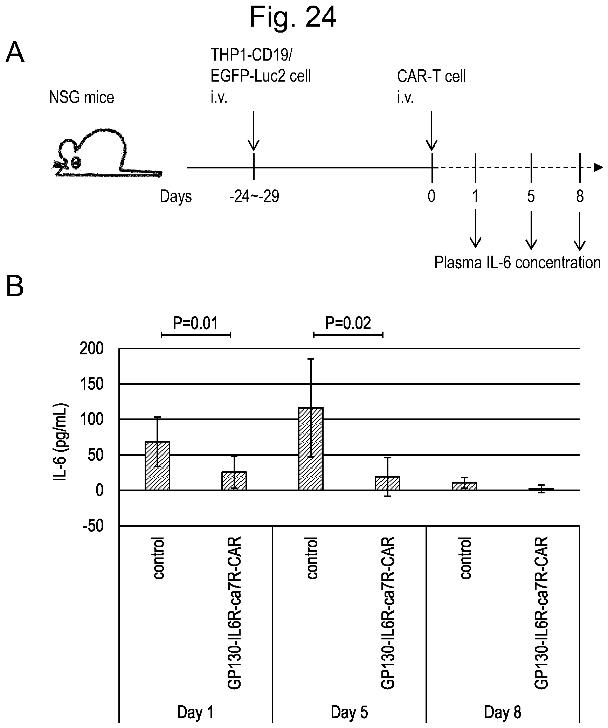

[Figure 24] Figure 24 shows measurement results of the ability of CAR-T cells co-transduced with GP130-IL6R-ca7R(M452L) and FMC63-28z to capture IL-6 produced from monocytic cells THP1-CD19/EGFP-Luc2 *in vivo.* Figure 24A shows the experimental procedure. Figure 24B shows IL-6 concentration in the plasma on one, five, and eight days after administration of the CAR-T cells. The results show mean values of experiments for n = 8, and error bars indicate standard deviation. P values shown in the figure indicate statistical significance by Student's t-test.

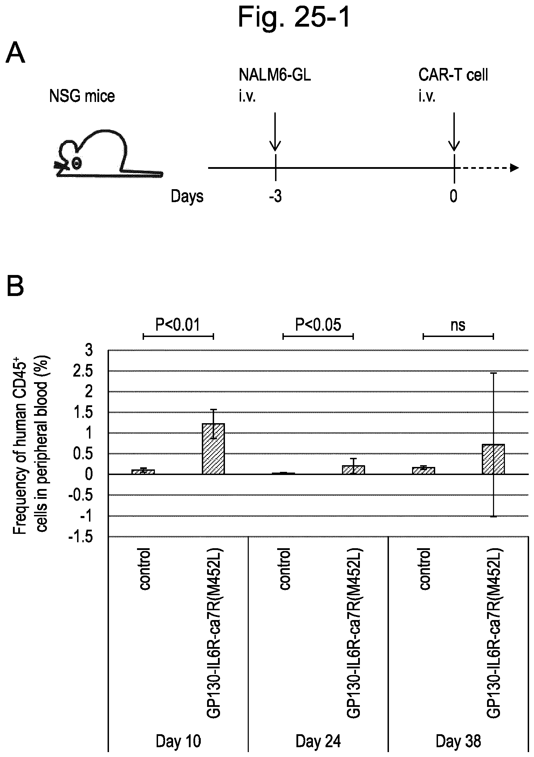

[Figure 25-1] Figure 25-1 shows antitumor effects of CAR-T cells co-transduced with GP130-IL6R-ca7R(M452L) in an *in vivo* leukemia model. Figure 25-1A shows the experimental procedure. Figure 25-1B shows the proportion of human CD45-positive T cells measured by flow cytometry in the peripheral blood collected on 10 to 38 days after administration of the CAR-T cells. The results show mean values of experiments for n = 2 to 7, and error bars indicate standard deviation. P values shown in the figure indicate statistical significance by Student's t-test, and "ns" indicates that no significant difference was detected.

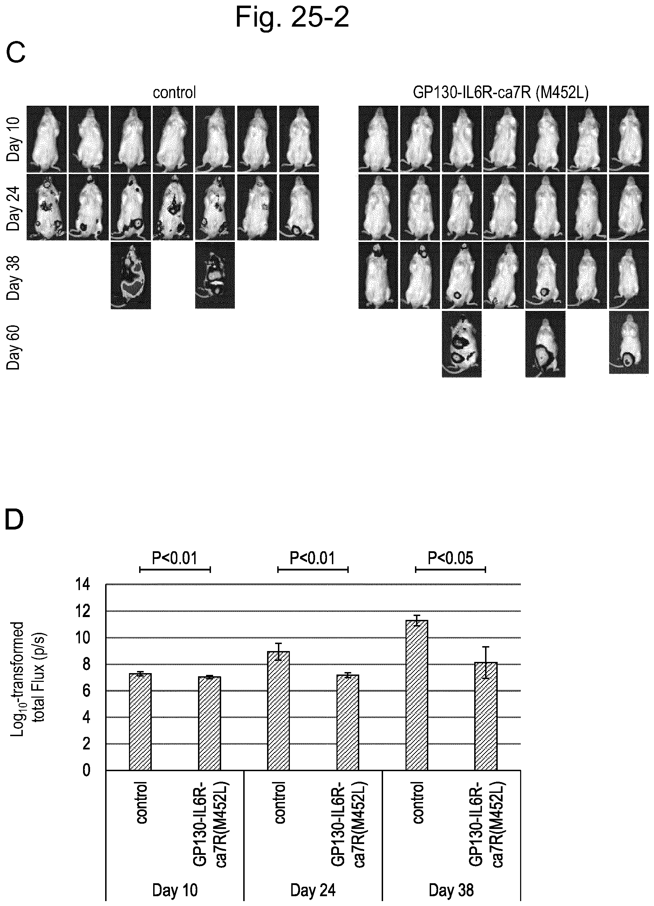

[Figure 25-2] Figure 25-2 is a continuation of Figure 25-1. Figure 25-2C shows results of measuring the volume of NALM6 tumor as the amount of luciferase luminescence by IVIS Imaging on 10, 24, 38, and 60 days after administration of the CAR-T cells. Figure 25-2D shows quantification results by logarithmically transforming the amount of luciferase luminescence. The results show mean values of experiments for n = 7, and error bars indicate standard deviation. P values shown in the figure indicate statistical significance by Student's t-test.

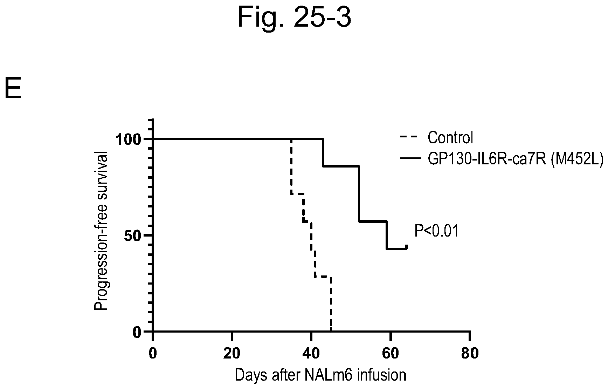

[Figure 25-3] Figure 25-3 is a continuation of Figure 25-2. Figure 25-3E shows results of analyzing the proportion of progression-free survival from the day of NALM6-GL cell transplantation. P values shown in the figure indicate statistical significance by Log-rank test.

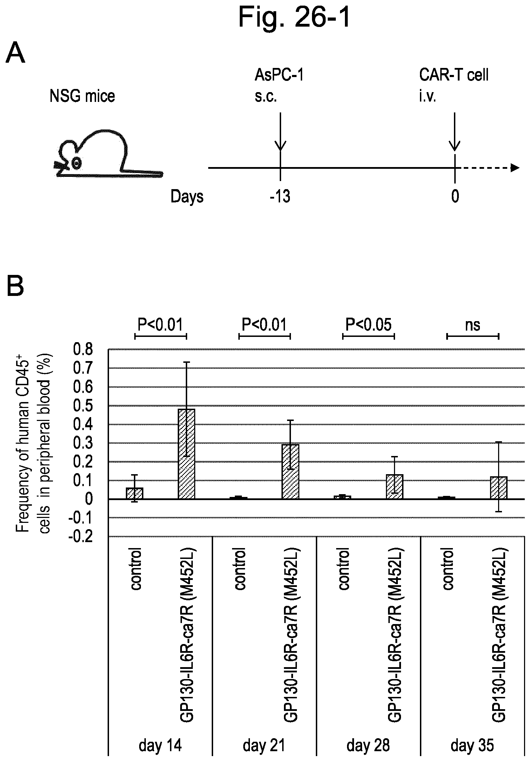

[Figure 26-1] Figure 26-1 shows antitumor effects of second-generation CAR-T cells co-transduced with GP130-IL6R-ca7R(M452L) in an *in vivo* solid tumor model (NSG mice subcutaneously implanted with mesothelin-positive pancreatic cancer cell line AsPC-1). Figure 26-1A shows the experimental procedure. Figure 26-1B shows the proportion of human CD45-positive T cells measured by flow cytometry in the peripheral blood collected on 14 to 35 days after administration of the CAR-T cells. The results show mean values of experiments for n = 7, and error bars indicate standard deviation. P values shown in the figure indicate statistical significance by Student's t-test, and "ns" indicates that no significant difference was detected.

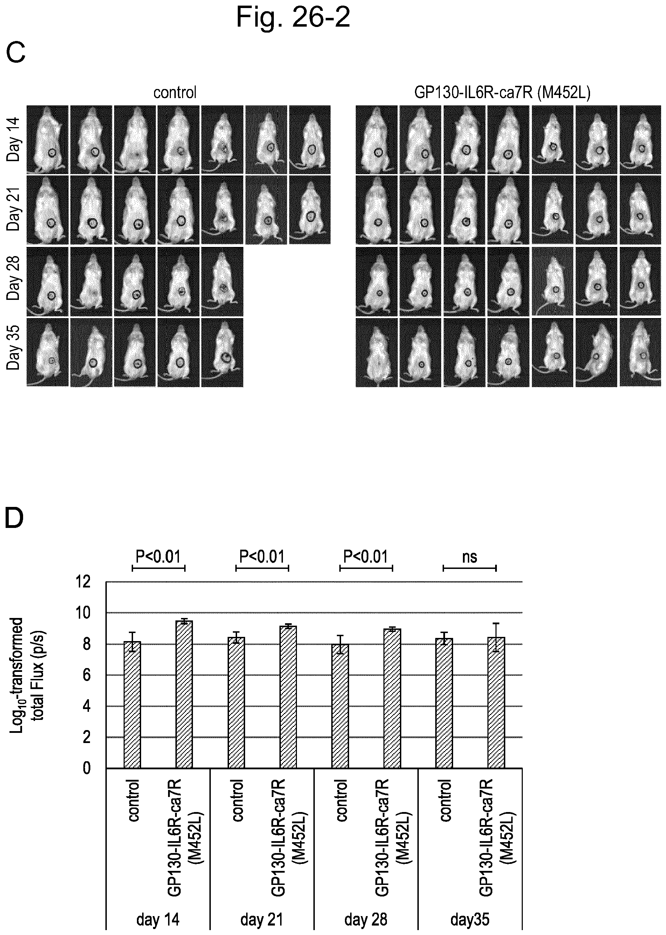

[Figure 26-2] Figure 26-2 is a continuation of Figure 26-1. Figure 26-2C shows results of measuring the second-generation CAR-T cells that have infiltrated into subcutaneous tumors, the amount of luciferase luminescence, by IVIS Imaging on 14 to 35 days after administration of the second-generation CAR-T cells. Figure 26-2D shows quantification results by logarithmically transforming the amount of luciferase luminescence. Note that in Figure 26-2, unlike Figure 25-2, CAR-T cells, but not tumor cells, are labeled with luciferase. The results show mean values of experiments for n = 7, and error bars indicate standard deviation. P values shown in the figure indicate statistical significance by Student's t-test, and "ns" indicates that no significant difference was detected.

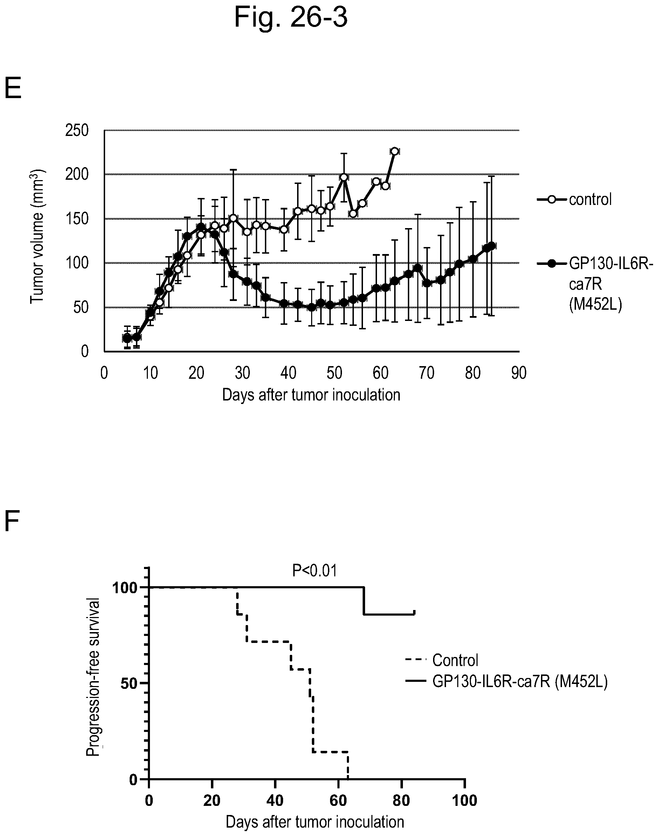

[Figure 26-3] Figure 26-3 is a continuation of Figure 26-2. Figure 26-3E shows the change in tumor volume over time after subcutaneous transplantation of AsPC-1 cells. The results show mean values of experiments for n = 7, and error bars indicate standard deviation. Figure 26-3F shows results of analyzing the percentage of progression-free survival from the day of AsPC-1 cell transplantation. P values shown in the figure indicate statistical significance by Log-rank test.

### Description of Embodiments

### 1. Chimeric cytokine receptor

### 1-1. Summary

The first aspect of the present invention is a chimeric cytokine receptor. The chimeric cytokine receptor of the present invention comprises a ligand-binding region at the N-terminal side and a T-cell activating region at the C-terminal side, and the T-cell activating region comprises the transmembrane domain and the intracellular domain of an IL-7 (Interleukin-7) receptor α chain. The ligand-binding region at the N-terminal side consists of a cytokine-binding region of a cytokine receptor such as an IL-6 receptor, an IL-1 receptor type 2, a GM-CSF receptor α chain, or a GM-CSF receptor β chain. When the chimeric cytokine receptor of the present invention is introduced into an immune cell such as a T cell, the immune cell can be provided with an activity of capturing an extracellular cytokine, and also can be activated.

### 1-2. Definition

The following terms frequently used herein are defined.

The term "adoptive immunotherapy" refers to a therapeutic method which comprises collecting cells such as immune cells from a donor, carrying out *e.g*., culture, stimulation, manipulation, and proliferation for the cells *in vitro,* and then introducing the cells into a recipient. For example, the cytotoxic activity of immune cells against cancer cells can be enhanced in a patient with cancer. Examples of adoptive immunotherapy include tumor infiltrating lymphocyte therapy, T-cell receptor-T (TCR-T) cell therapy, and chimeric antigen receptor-T (CAR-T) cell therapy. The adoptive immunotherapy may include leukapheresis. The adoptive immunotherapy mainly refers to autologous transplantation, in which the donor who provides the cells and the recipient into which the cells are introduced are the same individual, but also encompasses allogeneic transplantation (homoplastic or xenogeneic transplantation), in which the donor and recipient are different individuals.

The term "tumor infiltrating lymphocyte therapy" refers to a therapeutic method which comprises collecting tumor-infiltrating lymphocytes (TILs) from a donor, carrying out *e.g.,* activation or amplification *in vitro,* and then introducing the TILs into a recipient.

The term "T-cell receptor-T (TCR-T) cell therapy " refers to a therapeutic method which comprises introducing a cancer antigen-specific T-cell receptor (TCR) gene into immune cells such as T cells collected from the peripheral blood of a doner and then into a recipient.

The term "chimeric antigen receptor-T cell therapy (CAR-T cell therapy)" refers to a therapeutic method which comprises introducing a chimeric antigen receptor (CAR) gene into immune cells such as T cells collected from the peripheral blood of a donor to produce CAR-T cells that can recognize and attack cancer cells, followed by introducing the CAR-T cells into a recipient, *e.g*., by infusion. Currently, the CAR-T cell therapy is approved for B-cell hematological malignancies such as leukemia and lymphoma and expected to be a breakthrough cancer therapy technology after immune checkpoint blockade therapy.

The term "chimeric antigen receptor (CAR)" means a fusion protein comprising an extracellular domain capable of binding to an antigen such as a cancer antigen, a transmembrane domain, and an intracellular domain having signal transduction activity. More specifically, the chimeric antigen receptor is an artificial antigen receptor composed of a single-chain antibody (scFv) in which variable regions (V_{L} and V_{H}) of light and heavy chains derived from a monoclonal antibody recognizing a cancer cell surface antigen are linked, combined with a transmembrane domain, and a signaling domain of a co-stimulatory molecule involved in T cell activation. CARs can be roughly classified into first to third generations based on the structure and type of signaling domains contained in the CARs. A first-generation CAR has a signaling domain composed only of CD3ζ (also known as CD247). A second-generation CAR has a signaling domain containing one co-stimulatory molecule such as CD28 and CD137 (4-lBB) in addition to Cd3ζ. Representative examples of the second-generation CAR include a FMC63-28z CAR, which contains a portion of CD28 as a co-stimulatory molecule, and a FMC63-BBz CAR, which contains a portion of 4-lBB as a co-stimulatory molecule. The FMC63-28z CAR and the FMC63-BBz CAR both contain an anti-CD19 scFv as an scFv. A third-generation CAR has a signaling domain containing a plurality of co-stimulatory molecules such as CD28 and CD137 (4-1BB) in addition to Cd3ζ. In CAR-expressing T cells, a CAR can recognize tumor cells and activate the T cells, thereby efficiently killing the tumor cells. T cells transduced with a CAR gene are referred to as "chimeric antigen receptor-T cells (CAR-T cells)."

The term "cytokine release syndrome" refers to a symptom or pathology caused by the release of cytokines, such as inflammatory cytokines. The cytokine release syndrome may develop with administration of a drug such as an antibody drug or CAR-T cell therapy. In the CAR-T cell therapy, cytokines such as IL-6 are released from monocytes, macrophages, and other cells activated by CAR-T cells, and the cytokines induce fever and increased vascular permeability, which may result in a decrease in blood pressure and multiple organ dysfunction syndrome. Seventy-seven percent of patients receiving CAR-T cell therapy for B-cell acute lymphoblastic leukemia developed cytokine release syndrome (Maude S.L., et al., N Engl J Med, 2018, 378(5):439-448.).

The term "neurotoxicity syndrome" refers to a symptom or pathology which is based on neurotoxicity caused by administration of a drug or other substance. In the CAR-T cell therapy, cytokines such as IL-1β are released from monocytes, macrophages, and other cells activated by CAR-T cells, resulting in neurotoxicity that may cause, *e.g*., aphasia, mental abnormalities, convulsions, and cerebral edema. About 40% of patients receiving CAR-T cell therapy for B-cell acute lymphoblastic leukemia developed neurotoxicity syndrome (Maude S.L., et al., N Engl J Med, 2018, 378(5):439-448.).

"Cytokine" is a general term for a protein having a relatively small molecular weight secreted by cells. The cytokine is secreted mainly by immune cells and is responsible for transmission of information between cells. Examples of the cytokine include an interleukin, an interferon, a chemokine, a hematopoietic factor, a cell growth factor, and a tumor necrosis factor. Specific examples of the interleukin include IL-1, IL-6, and IL-7, as described below. Specific examples of the hematopoietic factor include GM-CSF described below.

"IL-1 (Interleukin-1)" is known as an inflammatory cytokine. IL-1 comprises IL-1α and IL-1β. When simply referred to herein as "IL-1," it is intended to include IL-1 derived from any species. Specific examples of the IL-1 include human IL-1 which comprises human IL-1α and human IL-1β. In neurotoxicity syndrome, IL-1β may be released from monocytes, macrophages, and other cells.

"IL-6 (Interleukin-6)" is known as an inflammatory cytokine. IL-6 is produced by T cells, macrophages, and other cells. When simply referred to herein as "IL-6," it is intended to include IL-6 derived from any species. Specific examples of the IL-6 include human IL-6. In cytokine release syndrome, IL-6 may be released from monocytes, macrophages, and other cells.

"IL-7 (Interleukin-7)" is known as a hematopoietic growth factor. IL-7 is secreted from stromal cells and other cells. When simply referred to herein as "IL-7," it is intended to include IL-7 derived from any species. Specific examples of the IL-7 include human IL-7.

A "Granulocyte macrophage colony-stimulating factor (GM-CSF)", also known as CSF2, is a cytokine that functions, for example, as a hematopoietic growth factor, and immunomodulatory factor. The GM-CSF is secreted by T cells, macrophages, NK cells, and other cells. When simply referred to herein as "GM-CSF", it is intended to include GM-CSF derived from any species. Specific examples of the GM-CSF include human GM-CSF.

A "cytokine receptor" is a receptor to which a cytokine binds as a ligand. Specific examples of the cytokine receptor include an interleukin receptor, an interferon receptor, a chemokine receptor, a hematopoietic factor receptor, a cell growth factor receptor, and a tumor necrosis factor receptor. Specific examples of the interleukin receptor include an IL-1 receptor, an IL-6 receptor, an IL-7 receptor α chain, gp130, and a common γ chain, as described below, and additionally, an IL-2 receptor, an IL-3 receptor, an IL-4 receptor, an IL-5 receptor, an IL-9 receptor, an IL-11 receptor, an IL-12 receptor, an IL-13 receptor, an IL-15 receptor, an IL-21 receptor, an IL-23 receptor, an IL-27 receptor, an IL-10 receptor, an IL-20 receptor, an IL-22 receptor, and an IL-28 receptor. Specific examples of the hematopoietic factor receptor include a GM-CSF receptor, as described below. Specific examples of the cell growth factor receptor include a TGF-β receptor. Specific examples of the tumor necrosis factor receptor include a TNF- α receptor (*e.g.,* type 1 and type 2), and a FAS receptor.

The "IL-1 receptor (Interleukin-1 receptor)" is a receptor to which IL-1 binds as a ligand, and IL-1 receptor type 1 (also referred to herein as IL1R1) and IL-1 receptor type 2 (also referred to herein as IL1R2) are known.

The "IL-1 receptor type 1" includes wild-type and mutant IL-1 receptor type 1 derived from any species. Examples thereof include a human IL-1 receptor type 1 consisting of the amino acid sequence of SEQ ID NO: 7 and an ortholog thereof. Examples of the mutant IL-1 receptor type 1 or IL-1 receptor type 1 ortholog include an IL-1 receptor type 1 consisting of an amino acid sequence in which one or more amino acids are deleted, substituted, or added in the amino acid sequence of SEQ ID NO: 7 or an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 7.

An "IL-1 receptor type 1 gene" is a gene encoding an IL-1 receptor type 1. Specific examples of the IL-1 receptor type 1 gene include an IL-1 receptor type 1 gene encoding the IL-1 receptor type 1 consisting of the amino acid sequence of SEQ ID NO: 7, such as a human IL-1 receptor type 1 gene consisting of the base sequence of SEQ ID NO: 8. Also included is an IL-1 receptor type 1 gene consisting of a base sequence in which one or more bases are deleted, substituted, or added in the base sequence of SEQ ID NO: 8 or a base sequence having 60% or more, 70% or more, 80% or more, 85% or more, 90%, 95% or more, 97% or more, 98% or more, or 99% or more base identity to the base sequence of SEQ ID NO: 8.

The "IL-1 receptor type 2" includes wild-type and mutant IL-1 receptor type 2 derived from any species. Examples thereof include a human IL-1 receptor type 2 consisting of the amino acid sequence of SEQ ID NO: 9 and an ortholog thereof. Examples of the mutant IL-1 receptor type 2 or IL-1 receptor type 2 ortholog include an IL-1 receptor type 2 consisting of an amino acid sequence in which one or more amino acids are deleted, substituted, or added in the amino acid sequence of SEQ ID NO: 9 or an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 9. In the human IL-1 receptor type 2, the extracellular domain is included in a region located at the N-terminal side of the transmembrane domain after signal peptide cleavage, for example, at positions 14 to 343 in the amino acid sequence of SEQ ID NO: 9. In the human IL-1 receptor type 2, the cytokine-binding region is included in the extracellular domain and is a region capable of binding to a cytokine, for example, at positions 14 to 343 or 14 to 296 in the amino acid sequence of SEQ ID NO: 9.

An "IL-1 receptor type 2 gene" is a gene encoding an IL-1 receptor type 2. Specific examples of the IL-1 receptor type 2 gene include an IL-1 receptor type 2 gene encoding the IL-1 receptor type 2 consisting of the amino acid sequence of SEQ ID NO: 9, such as a human IL-1 receptor type 2 gene consisting of the base sequence of SEQ ID NO: 10. Also included is an IL-1 receptor type 2 gene consisting of a base sequence in which one or more bases are deleted, substituted, or added in the base sequence of SEQ ID NO: 10 or a base sequence having 60% or more, 70% or more, 80% or more, 85% or more, 90%, 95% or more, 97% or more, 98% or more, or 99% or more base identity to the base sequence of SEQ ID NO: 10.

The "IL-6 receptor (Interleukin-6 receptor)" is a receptor to which IL-6 binds as a ligand. The IL-6 receptor is also known as an IL-6 receptor α subunit or CD 126 and referred to as IL6R or IL6RA. The IL-6 receptor binds to IL-6 together with gp130 (Glycoprotein 130) described below to form a heterotrimer consisting of the IL-6, IL-6 receptor, and gp130. The IL-6 receptor includes wild-type and mutant IL-6 receptor derived from any species. Examples thereof include a human IL-6 receptor consisting of the amino acid sequence of SEQ ID NO: 11 and an ortholog thereof. Examples of the mutant IL-6 receptor or IL-6 receptor ortholog include an IL-6 receptor consisting of an amino acid sequence in which one or more amino acids are deleted, substituted, or added in the amino acid sequence of SEQ ID NO: 11 or an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 11. In the human IL-6 receptor, the extracellular domain is included in a region located at the N-terminal side of the transmembrane domain after signal peptide cleavage, for example, at positions 21 to 365 in the amino acid sequence of SEQ ID NO: 11. In the human IL-6 receptor, the cytokine-binding region is included in the extracellular domain and is a region capable of binding to a cytokine, for example, at positions 21 to 365 or 110 to 338 in the amino acid sequence of SEQ ID NO: 11.

An "IL-6 receptor gene" is a gene encoding an IL-6 receptor. Specific examples of the IL-6 receptor gene include an IL-6 receptor gene encoding the IL-6 receptor consisting of the amino acid sequence of SEQ ID NO: 11, such as a human IL-6 receptor gene consisting of the base sequence of SEQ ID NO: 12. Also included is an IL-6 receptor gene consisting of a base sequence in which one or more bases are deleted, substituted, or added in the base sequence of SEQ ID NO: 12 or a base sequence having 60% or more, 70% or more, 80% or more, 85% or more, 90%, 95% or more, 97% or more, 98% or more, or 99% or more base identity to the base sequence of SEQ ID NO: 12.

"gp130 (Glycoprotein 130)" is also known as IL6ST or CD130. The gp130 includes wild-type and mutant gp130 derived from any species. Examples thereof include human gp130 consisting of the amino acid sequence of SEQ ID NO: 13 and an ortholog thereof. Examples of the mutant gp130 or gp130 ortholog include an gp130 consisting of an amino acid sequence in which one or more amino acids are deleted, substituted, or added in the amino acid sequence of SEQ ID NO: 13 or an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 13. In the human gp130, the extracellular domain is included in a region located at the N-terminal side of the transmembrane domain after signal peptide cleavage, for example, at positions 23 to 619 in the amino acid sequence of SEQ ID NO: 13. In the human gp130, the cytokine-binding region is included in the extracellular domain, the region capable of binding to a cytokine, and consists of, for example, positions 23 to 326 in the amino acid sequence of SEQ ID NO: 13.

A "gp130 gene" is a gene encoding gp130. Specific examples of the gp130 gene include a gp130 gene encoding the gp130 consisting of the amino acid sequence of SEQ ID NO: 13, such as a human gp130 gene consisting of the base sequence of SEQ ID NO: 14. Also included is an gp130 gene consisting of a base sequence in which one or more bases are deleted, substituted, or added in the base sequence of SEQ ID NO: 14 or a base sequence having 60% or more, 70% or more, 80% or more, 85% or more, 90%, 95% or more, 97% or more, 98% or more, or 99% or more base identity to the base sequence of SEQ ID NO: 14.

The "IL-7 receptor (Interleukin-7 receptor)" is a receptor to which IL-7 binds as a ligand, and is a heterodimer consisting of an IL-7 receptor α chain and a common γ chain (also known as a yc chain or CD132). Major signal transduction pathways activated by the binding of IL-7 to the IL-7 receptor include a Jak-Stat pathway and a PI3K-Akt pathway. When IL-7 binds to the IL-7 receptor, a Jak kinase is phosphorylated, followed by phosphorylation of tyrosine residues (such as Y401, Y449, and Y456) in the IL-7 receptor α chain. Of these, the phosphorylated tyrosine residue at position 449 (pY449) is known to be particularly important (Jiang Q, et al., Mol Cell Biol., 2004, 24(14):6501-13.). pY449 functions as a docking site for a SH2 domain protein containing a Stat family transcription factor activated by phosphorylation by Jak. A SOCS family protein is also known to be involved in IL-7 receptor signaling. The IL-7 receptor signaling in a lymphocyte is known to lead to survival, proliferation, and differentiation of the lymphocyte depending on the stage of development.

The "IL-7 receptor α chain" is also known as CD 127 and referred to herein as IL7RA or IL7Rα. The IL-7 receptor α chain includes wild-type and mutant IL-7 receptor α chain derived from any species. Examples thereof include a human IL-7 receptor α chain consisting of the amino acid sequence of SEQ ID NO: 1 and an ortholog thereof. Examples of the mutant IL-7 receptor α chain or IL-7 receptor α-chain ortholog include an IL-7 receptor α chain consisting of an amino acid sequence in which one or more amino acids are deleted, substituted, or added in the amino acid sequence of SEQ ID NO: 1 and an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 1. In the human IL-7 receptor α chain, the extracellular domain may be included in a region located at the N-terminal side of the transmembrane domain after signal peptide cleavage, for example, at positions 21 to 239 in the amino acid sequence of SEQ ID NO: 1. The transmembrane domain in the human IL-7 receptor α chain may be at positions 240 to 264 in the amino acid sequence of SEQ ID NO: 1. In the human IL-7 receptor α chain, the intracellular domain may be included at positions 265 to 459 in the amino acid sequence of SEQ ID NO: 1. The intracellular domain of the human IL-7 receptor α chain contains several functional motifs, including a JAK-binding motif (also known as a "BOX1 motif'), a STAT3 association motif, and a STAT5/PI3K association motif (also known as a "SH2 domain binding motif") (Figure 1). In the human IL-7 receptor α chain consisting of the amino acid sequence of SEQ ID NO: 1, the JAK-binding motif is at positions 272 to 280 (Val-Trp-Pro-Ser-Leu-Pro-Asp-His-Lys sequence, SEQ ID NO: 15), the STAT3 association motif is at positions 456 to 459 (Tyr-Gln-Asn-Gln sequence, SEQ ID NO: 16), and the STAT5/PI3K association motif is at positions 449 to 452 (Tyr-Val-Thr-Met sequence, SEQ ID NO: 17).

A constitutively active mutation is known in the IL-7 receptor α chain. A "constitutively active IL-7 receptor α chain" or "IL-7 receptor α chain having a constitutively active mutation" is a mutant of the IL-7 receptor α chain that can be activated even under conditions where IL-7 is not bound as a ligand. In a cell expressing the constitutively active IL-7 receptor α chain, at least some of the signal transduction pathways downstream of the IL-7 receptor α chain can be activated even in the absence of IL-7. Known constitutively active insertion mutations include an insertion of an amino acid sequence consisting of Pro-Pro-Cys-Leu (SEQ ID NO: 2) between position 243 and position 244 in the amino acid sequence of SEQ ID NO: 1, an insertion of an amino acid sequence consisting of Phe-Ser-Cys-Gly-Pro (SEQ ID NO: 3) between position 241 and position 242 in the amino acid sequence of SEQ ID NO: 1, an insertion of an amino acid sequence consisting of Cys-His-Leu (SEQ ID NO: 4) between position 244 and position 245 in the amino acid sequence of SEQ ID NO: 1, an insertion of an amino acid sequence consisting of Pro-Pro-Val-Cys-Ser-Val-Thr (SEQ ID NO: 5) between position 244 and position 245 in the amino acid sequence of SEQ ID NO: 1, and an insertion of an amino acid sequence consisting of Lys-Cys-His (SEQ ID NO: 6) between position 246 and position 247 in the amino acid sequence of SEQ ID NO: 1.

In addition, the functional motifs described above in the intracellular domain of the IL-7 receptor α chain are also known to have a mutation which reduces the activity thereof (Figure 13A). Examples of a mutation that reduces the activity of the STAT5/PI3K association motif include a Y449F mutation that replaces a Tyr residue at position 449 with a Phe residue in the amino acid sequence of SEQ ID NO: 1 and a M452L mutation that replaces a Met residue at position 452 with a Leu residue in the amino acid sequence of SEQ ID NO: 1. Examples of a mutation that reduces the activity of the STAT3 association motif include a Y456F mutation that replaces a Tyr residue at position 456 with a Phe residue in the amino acid sequence of SEQ ID NO: 1.

An "IL-7 receptor α-chain gene" is a gene encoding an IL-7 receptor α chain. Specific examples of the IL-7 receptor α-chain gene include an IL-7 receptor α-chain gene encoding the IL-7 receptor α chain consisting of the amino acid sequence of SEQ ID NO: 1, such as a human IL-7 receptor α-chain gene consisting of the base sequence of SEQ ID NO: 19. Also included is an IL-7 receptor α-chain gene consisting of a base sequence in which one or more bases are deleted, substituted, or added in the base sequence of SEQ ID NO: 19 or a base sequence having 60% or more, 70% or more, 80% or more, 85% or more, 90%, 95% or more, 97% or more, 98% or more, or 99% or more base identity to the base sequence of SEQ ID NO: 19.

The "Granulocyte macrophage colony-stimulating factor receptor (GM-CSF receptor)" is a receptor to which the GM-CSF binds as a ligand, and is a heterodimer composed of a GM-CSF receptor α chain and a GM-CSF receptor β chain.

The "GM-CSF receptor α chain" is also known as CD116 and referred to herein as CSF2RA. The GM-CSF receptor α chain includes wild-type and mutant GM-CSF receptor α chain derived from any species. Examples thereof include a human GM-CSF receptor α chain consisting of the amino acid sequence of SEQ ID NO: 20 and an ortholog thereof. Examples of the mutant GM-CSF receptor α chain or GM-CSF receptor α-chain ortholog include a GM-CSF receptor α chain consisting of an amino acid sequence in which one or more amino acids are deleted, substituted, or added in the amino acid sequence of SEQ ID NO: 20 and an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 20. In the human GM-CSF receptor α chain, the extracellular domain is included in a region located at the N-terminal side of the transmembrane domain after signal peptide cleavage, for example, at positions 23 to 320 in the amino acid sequence of SEQ ID NO: 20. In the human GM-CSF receptor α chain, the cytokine-binding region is included in the extracellular domain, the region capable of binding to a cytokine, and is, for example, a region consisting of positions 25 to 320 in the amino acid sequence of SEQ ID NO: 20 or a full-length extracellular domain consisting of positions 23 to 320 in the amino acid sequence of SEQ ID NO: 20.

A "GM-CSF receptor α-chain gene" is a gene encoding a GM-CSF receptor α chain. Specific examples of the GM-CSF receptor α-chain gene include a GM-CSF receptor α-chain gene encoding a GM-CSF receptor α chain consisting of the amino acid sequence of SEQ ID NO: 20, such as a human GM-CSF receptor α-chain gene consisting of a base sequence of SEQ ID NO: 21. Also included is an GM-CSF receptor α-chain gene having a base sequence in which one or more bases are deleted, substituted, or added in the base sequence of SEQ ID NO: 21 or a base sequence having 60% or more, 70% or more, 80% or more, 85% or more, 90%, 95% or more, 97% or more, 98% or more, or 99% or more base identity to the base sequence of SEQ ID NO: 21.

The "GM-CSF receptor β chain" includes wild-type and mutant GM-CSF receptor β chain derived from any species. Examples thereof include a human GM-CSF receptor β chain consisting of the amino acid sequence of SEQ ID NO: 22 and an ortholog thereof. Examples of the mutant GM-CSF receptor β chain or GM-CSF receptor β-chain ortholog include a GM-CSF receptor β chain consisting of an amino acid sequence in which one or more amino acids are deleted, substituted, or added in the amino acid sequence of SEQ ID NO: 22 and an amino acid sequence having 60% or more, 70% or more, 80% or more, 85% or more, 90% or more, 95% or more, 96% or more, 97% or more, 98% or more, or 99% or more identity to the amino acid sequence of SEQ ID NO: 22. In the human GM-CSF receptor β chain, the extracellular domain is included in a region located at the N-terminal side of the transmembrane domain after signal peptide cleavage, for example, at positions 17 to 443 in the amino acid sequence of SEQ ID NO: 22. In the human GM-CSF receptor β chain, the cytokine-binding region is included in the extracellular domain and is a region capable of binding to a cytokine, for example, at positions 17 to 132 and/or 339 to 436 in the amino acid sequence of SEQ ID NO: 22.

A "GM-CSF receptor β-chain gene" is a gene encoding a GM-CSF receptor β chain. Specific examples of the GM-CSF receptor β-chain gene include a GM-CSF receptor β-chain gene encoding a GM-CSF receptor β chain consisting of the amino acid sequence of SEQ ID NO: 22, such as a human GM-CSF receptor β-chain gene consisting of the base sequence of SEQ ID NO: 23. Also included is an GM-CSF receptor β-chain gene having a base sequence in which one or more bases are deleted, substituted, or added in the base sequence of SEQ ID NO: 23 or a base sequence having 60% or more, 70% or more, 80% or more, 85% or more, 90%, 95% or more, 97% or more, 98% or more, or 99% or more base identity to the base sequence of SEQ ID NO: 23.

As used herein, the term "chimeric cytokine receptor" means a fusion protein containing a portion derived from two or more cytokine receptors.

As used herein, an "immune cell" includes a cell type that can function as part of the immune system, as well as an undifferentiated (immature) cell and a progenitor cell that may differentiate into such a cell type (*e.g.,* an immune progenitor cell). Specific examples of the immune cell include a lymphocyte, a granulocyte, a dendritic cell, a macrophage, and a monocyte. Examples of the lymphocyte include a T cell, a B cell, and a natural killer cell (NK cell). The lymphocyte may be a tumor infiltrating lymphocyte. Examples of the T cell include a killer T cell (cytotoxic T cell), a helper T cell, and a regulatory T cell. The T cell may be either a CD8-positive T cell or a CD4-positive T cell. The T cell can also be classified into a naive T cell, a memory T cell, and an effector T cell, any of which may be used. It is known that the naive T cell and the memory T cell are abundantly contained in peripheral blood, whereas the effector T cell is scarce in peripheral blood. Examples of the granulocyte include a neutrophil, an eosinophil, and a basophil. Although immune cells may not include stem cells in a narrow sense, stem cells that can differentiate into lymphocytes (*e.g*., hematopoietic stem cells) are also included herein.

The term "signal peptide" refers to an extracellular localization signal, also called a signal peptide, that is required for extracellular secretion of a protein biosynthesized by gene expression. A signal sequence may include a region composed of hydrophobic amino acids. The signal peptide is cleaved off by a signal peptidase after translation and before extracellular translocation. The signal peptide sequence is present at the N-terminus of many secretory proteins and membrane proteins and has a length of, for example, 15 to 30 amino acids. The signal peptide may be derived from any species, which may be derived from human or non-human, such as an insect cell and a virus, but is preferably of human origin. Specific examples of the signal peptide include a signal peptide derived from human Oncostatin M.

The term "linker peptide" refers to a peptide that can be inserted between fused portions of a fusion protein, such as the chimeric cytokine receptor of the present invention, to allow each of the portions to exert its desired function. Although the length of the linker peptide is not limited, it is usually 3 to 100 amino acids in length and preferably 5 to 50 amino acids in length, for example. Peptides containing many amino acids with relatively small side chains, such as serine and glycine, are often used.

The term "tag peptide" refers to a short peptide consisting of tens of amino acids to several tens of amino acids that can label a protein and is used for protein detection and purification. Usually, a base sequence encoding a tag peptide is linked to the 5'- or 3'- terminal side of a gene encoding a protein to be labeled to express it as a fusion protein with the tag peptide for labeling. While various tag peptides have been developed in the field, any tag peptide may be used. Specific examples of the tag peptide include FLAG, HA, His, PA, and myc.

As used herein, the term "capture" refers to binding of a ligand such as a cytokine by a ligand-binding region contained in a chimeric cytokine receptor or a fragment thereof. When a cytokine is captured, for example, by a chimeric cytokine receptor, the function of the cytokine, such as signaling via an endogenous cytokine receptor, may be suppressed or inhibited. The cytokines captured by a chimeric cytokine receptor on the cell membrane can be absorbed into the cell.

As used herein, the term "plurality" refers to an integer of 2 or more, for example, an integer of 2 to 10, 2 to 7, 2 to 5, 2 to 4, or 2 to 3.

As used herein, the term "amino acid identity (amino acid sequence identity)" refers to the proportion (%) of the number of matched amino acid residues in the total number of amino acid residues when the amino acid sequences of two polypeptides to be compared are aligned by inserting a gap, if necessary, into one or both of them so that the number of the matched amino acid residues is maximized. The term "base identity (base sequence identity)" can be determined in the same manner.

As used herein, the term "amino acid substitution" refers to substitution among 20 types of amino acids constituting a natural protein. The amino acid substitution is preferably within a group of conservative amino acids with similar properties such as charge, side chain, polarity, and aromaticity. Examples thereof include substitutions within the group of uncharged polar amino acids with a low polar side chain (Gly, Asn, Gln, Ser, Thr, Cys, and Tyr), the group of branched chain amino acids (Leu, Val, and Ile), the group of neutral amino acids (Gly, Ile, Val, Leu, Ala, Met, and Pro), the group of neutral amino acids with a hydrophilic side chain (Asn, Gln, Thr, Ser, Tyr, and Cys), the group of acidic amino acids (Asp and Glu), the group of basic amino acids (Arg, Lys, and His), and the group of aromatic amino acids (Phe, Tyr, and Trp).

### 1-3. Configuration

The chimeric cytokine receptor of the present invention includes a ligand-binding region at the N-terminal side and a T-cell activating region at the C-terminal side.

As used herein, the term "ligand-binding region" means a region that can bind with a ligand in the extracellular region of the chimeric cytokine receptor of the present invention. In the chimeric cytokine receptor of the present invention, the ligand-binding region consists of the cytokine-binding region of the cytokine receptor. The cytokine receptor from the cytokine binding region is, in principle, a cytokine receptor other than the IL-7 receptor α chain.

As used herein, the "cytokine-binding region" is a region that can bind with a cytokine. The cytokine to which the cytokine-binding region binds is not limited, and examples thereof includes IL-6, IL-1, and GM-CSF. As the cytokine-binding region constituting the ligand-binding region of the chimeric cytokine receptor of the present invention, a cytokine-binding region derived from a cytokine receptor other than the IL-7 receptor α chain can be used, for example, a cytokine-binding region of the IL-6 receptor, a cytokine-binding region of the IL-1 receptor type 2, a cytokine-binding region of the GM-CSF receptor α chain, and/or a cytokine-binding region of the GM-CSF receptor β chain.

The cytokine-binding region of the IL-6 receptor is included in the extracellular domain of the IL-6 receptor, the region capable of binding to a cytokine such as IL-6, and more specifically may be a region containing a D2 domain and/or a D3 domain, which are known to be important for IL-6 binding in the IL-6 receptor (Schwantner A., et al., J Biol Chem., 2004, 279(1):571-6; Yawata H., et al., EMBO J, 1993, 12(4):1705-1712.). Specific examples of the cytokine-binding region of the IL-6 receptor include a region consisting of positions 110 to 365 or positions 110 to 338 in the amino acid sequence of SEQ ID NO: 11 and a full-length extracellular region consisting of positions 21 to 365 in the amino acid sequence of SEQ ID NO: 11.

The cytokine-binding region of the IL-1 receptor type 2 is included in the extracellular domain of the IL-1 receptor type 2, and is a region capable of binding to a cytokine such as IL-1, and more specifically may be a region containing three IgC2 domains and a protease cleavage region in the IL-1 receptor type 2. Examples of such a region include an extracellular region of the IL-1 receptor type 2 (Liu C, et al., J Biol Chem, 1996, 271(34):20965-20972). Specific examples of the cytokine-binding region of the IL-1 receptor type 2 include a region consisting of positions 14 to 296 in the amino acid sequence of SEQ ID NO: 9 and a full-length extracellular region consisting of positions 14 to 343 in the amino acid sequence of SEQ ID NO: 9.

The cytokine-binding region of the GM-CSF receptor α chain is included in the extracellular domain of the GM-CSF receptor α chain, and is a region capable of binding to a cytokine such as GM-CSF, and more specifically may be a region containing a D1 domain (e.g., a region consisting of positions 25 to 113 in the amino acid sequence of SEQ ID NO: 20) and/or a D2-D3 domain (also known as a Class I cytokine receptor homology module, for example, a region consisting of positions 122 to 320 in the amino acid sequence of SEQ ID NO: 20), which are known to be important for GM-CSF binding in the GM-CSF receptor α chain (Mirza S., et al., Biochem J, 2010, 426(3):307-17; Hansen G., Cell, 2008, 134(3):496-507.). Specific examples of such a region include an extracellular region of the GM-CSF receptor α chain, such as an extracellular region consisting of positions 25 to 320 in the amino acid sequence of SEQ ID NO: 20.

The cytokine-binding region of the GM-CSF receptor β chain is included in the extracellular domain of the GM-CSF receptor β chain, and is a region capable of binding to a cytokine such as GM-CSF, and more specifically may be a region containing a D1 domain and/or a D4 domain, which are known to be important for GM-CSF binding in the GM-CSF receptor β chain. More preferred may be a region further containing D2 and/or a D3 domain (example of α-chain binding region in the GM-CSF receptor β chain: positions 133 to 240 and positions 241 to 338 in the amino acid sequence of SEQ ID NO: 22), which are known to be important for multimer formation of α and β chains (Hansen G., et al., Cell, 2008, 134(3):496-507; Haman A., et al., J Biol Chem, 1999, 274(48):34155-63.). Specific examples of such a region include an extracellular region containing positions 17 to 132 and/or positions 339 to 436 in the amino acid sequence of SEQ ID NO: 22, such as a full-length extracellular region consisting of positions 17 to 443 and a region consisting of positions 17 to 132 and/or positions 339 to 436.

In one embodiment, the cytokine receptor may include the cytokine-binding region of the IL-6 receptor and the ligand-binding region of gp130 (Glycoprotein 130). In this case, the ligand-binding region of gp130 may be located either at the N-terminal side or the C-terminal side of the cytokine-binding region of the IL-6 receptor but is preferably at the N-terminal side.

The ligand-binding region of gp130 is included in the extracellular domain of the gp130, and is a region capable of binding to a cytokine such as IL-6, and more specifically may be a region containing a D1 domain, a D2 domain, and/or a D3 domain, which are known to be important for IL-6 binding in the gp130 (Chow D., et al., Science, 2001, 291(5511):2150-5; Pflanz S., Biochem J, 2001, 356(Pt 2):605-12.). Specific examples of such a region include an extracellular region of gp130, such as an extracellular region consisting of positions 23 to 326 in the amino acid sequence of SEQ ID NO: 13.

As used herein, the term "T-cell activating region" refers to a region that can activate T cells in the chimeric cytokine receptor of the present invention. The T-cell activating region can activate functions such as T-cell proliferative ability and cytotoxic activity. The T-cell activating region of the chimeric cytokine receptor of the present invention includes the transmembrane domain and the intracellular domain of an IL-7 (Interleukin-7) receptor α chain.

As used herein, the term "transmembrane domain of the IL-7 receptor α chain" refers to a portion consisting of the amino acid sequence that penetrates the cell membrane in the IL-7 receptor α chain. The transmembrane domain of the IL-7 receptor α chain can be determined by a known method based on the amino acid sequence of the IL-7 receptor α chain. The transmembrane domain of the human IL-7 receptor α chain may be at positions 240 to 264 in the amino acid sequence of SEQ ID NO: 1.

As used herein, the term "intracellular domain of the IL-7 receptor α chain" refers to a portion consisting of the amino acid sequence located intracellularly in the IL-7 receptor α chain. The intracellular domain of the IL-7 receptor is a region included in the portion located at the C-terminal side more than the transmembrane domain of the IL-7 receptor α-chain described above. Examples thereof include a region containing a Box1 motif (e.g., positions 272 to 280 in the amino acid sequence of SEQ ID NO: 1) and/or an SH2 domain-binding motif (e.g., positions 449 to 452 in the amino acid sequence of SEQ ID NO: 1) in the amino acid sequence of the IL-7 receptor α chain, such as an intracellular region consisting of positions 265 to 459 in the amino acid sequence of SEQ ID NO: 1.

As used herein, the phrase "comprising the transmembrane domain and the intracellular domain of the IL-7 receptor α chain" refers to the inclusion of both the transmembrane domain of the IL-7 receptor α chain and the intracellular domain of the IL-7 receptor α chain described above. Specific examples of the T-cell activating region comprising the transmembrane domain and the intracellular domain of the IL-7 receptor α chain include a region containing positions 240 to 459 in the amino acid sequence of SEQ ID NO: 1.

The T-cell activating region in the chimeric cytokine receptor of the present invention preferably has a constitutively active mutation in the transmembrane domain of the IL-7 receptor. The constitutively active mutation may be a mutation that can activate T cells, and a constitutively active mutation known in the art can be used. The constitutively active mutation may be an insertion mutation. The constitutively active insertion mutation is known (Shochat C., et al., J Exp Med, 2011, 208(5):901-8). For example, the insertion can be selected from the group consisting of (a) to (e) below: (a) an insertion of an amino acid sequence consisting of Pro-Pro-Cys-Leu (SEQ ID NO: 2) between position 243 and position 244 in the amino acid sequence of SEQ ID NO: 1, (b) an insertion of an amino acid sequence consisting of Phe-Ser-Cys-Gly-Pro (SEQ ID NO: 3) between position 241 and position 242 in the amino acid sequence of SEQ ID NO: 1, (c) an insertion of an amino acid sequence consisting of Cys-His-Leu (SEQ ID NO: 4) between position 244 and position 245 in the amino acid sequence of SEQ ID NO: 1, (d) an insertion of an amino acid sequence consisting of Pro-Pro-Val-Cys-Ser-Val-Thr (SEQ ID NO: 5) between position 244 and position 245 in the amino acid sequence of SEQ ID NO: 1, and (e) an insertion of an amino acid sequence consisting of Lys-Cys-His (SEQ ID NO: 6) between position 246 and position 247 in the amino acid sequence of SEQ ID NO: 1.

The chimeric cytokine receptor of the present invention may have a mutation which reduces the activity of one or more motifs selected from the group consisting of a JAK-binding motif, a STAT3 association motif, and a STAT5/PI3K association motif which are contained in the intracellular domain of the IL-7 receptor α chain. By adding a mutation to the amino acid sequences constituting these motifs, it is possible to suppress only particular downstream signals, thereby enhancing therapeutic efficacy of the chimeric cytokine receptor of the present invention.

In one embodiment, the chimeric cytokine receptor of the present invention may have a mutation at the Y449 residue and/or the M452 residue in the STAT5/PI3K association motif contained in the intracellular domain of the IL-7 receptor α chain. The chimeric cytokine receptor of the present invention may also have a mutation at the Y456 residue in the STAT3 association motif contained in the intracellular domain of the IL-7 receptor α chain. The Y449 residue is known as an important residue involved in the recruitment of STAT3 and STATS proteins, and mutation of the Y449 residue can attenuate cell proliferation signals induced by IL-7 (Lin J.X., et al., Immunity 1995, 2(4):331-9; Corcoran, A.E., et al., EMBO J, 1996, 15(8): 1924-1932.). The M452 residue is known to be involved in the recruitment of PI3K proteins, and mutation of the M452 residue attenuates the PI3K-Akt signal while maintaining the STAT3/5 signal and thus does not affect IL-7-associated cell proliferation. Since excessive PI3K signals can promote T cell differentiation and reduce the long-term surviving ability, mutation of the M452 residue can promote the formation of memory T cells having a long-term surviving ability (Cui G., et al., J Immunol, 2020, 204(4):844-857). The Y456 residue is also known to have an attenuated proliferation signal upon mutation (Zhong J., et al., BMC Immunol, 2010, 11:5.). Specific examples of the mutation in Y449, M452, and/or Y456 include a Y449F mutation, an M452L mutation, and/or a Y456F mutation. In one embodiment, the chimeric cytokine receptor of the present invention has an M452L mutation in the STAT5/PI3K association motif contained in the intracellular domain of the IL-7 receptor α chain.

The chimeric cytokine receptor of the present invention may contain, for example, a signal peptide, linker peptide, and/or tag peptide, if necessary, in addition to the ligand-binding region and the T-cell activating region described above. If the ligand-binding region does not contain a signal peptide, the chimeric cytokine receptor of the present invention preferably contains a signal peptide at the N-terminal side of the ligand-binding region.

In one embodiment, the ligand-binding region of the chimeric cytokine receptor of the present invention is derived from the IL-6 receptor, and the T-cell activating region is derived from the IL-7 receptor α chain, an example of which includes a chimeric cytokine receptor consisting of the amino acid sequence of SEQ ID NO: 24.

In one embodiment, the ligand-binding region of the chimeric cytokine receptor of the present invention is derived from the IL-6 receptor, the T-cell activating region is derived from the IL-7 receptor α chain, and the chimeric cytokine receptor further includes a ligand-binding region of gp130 at the N-terminal side of the ligand-binding region derived from the IL-6 receptor, an example of which includes a chimeric cytokine receptor consisting of the amino acid sequence of SEQ ID NO: 25.

In one embodiment, the ligand-binding region of the chimeric cytokine receptor of the present invention is derived from the IL-1 receptor type 2, and the T-cell activating region is derived from the IL-7 receptor α chain, an example of which includes a chimeric cytokine receptor consisting of the amino acid sequence of SEQ ID NO: 26.

In one embodiment, the ligand-binding region of the chimeric cytokine receptor of the present invention is derived from the GM-CSF receptor α chain, and the T-cell activating region is derived from the IL-7 receptor α chain, an example of which includes a chimeric cytokine receptor consisting of the amino acid sequence of SEQ ID NO: 27.

In one embodiment, the ligand-binding region of the chimeric cytokine receptor of the present invention is derived from the GM-CSF receptor β chain, and the T-cell activating region is derived from the IL-7 receptor α chain, an example of which includes a chimeric cytokine receptor consisting of the amino acid sequence of SEQ ID NO: 28.

### 1-4. Effect

The chimeric cytokine receptor of the present invention can activate immune cells and capture extracellular cytokines, and based on these two functions, can achieve both improved therapeutic efficacy and reduced side effects of adoptive immunotherapy.

The ligand-binding region in the chimeric cytokine receptor of the present invention can reduce the activity of a cytokine such as IL-6, IL-1β, and GM-CSF by capturing extracellular cytokines, thereby suppressing or avoiding cytokine release syndrome and neurotoxicity syndrome associated with adoptive immunotherapy such as CAR-T cell therapy. Examples of more specific mechanisms of suppression or avoidance include, but are not limited to, the following: the ligand-binding region derived from the IL-6 receptor can mediate the absorption of IL-6 into cells. The ligand-binding region derived from the IL-1 receptor type 2 can be cleaved and released extracellularly as a soluble receptor to capture IL-1β and/or mediate the absorption of IL-1β into the cells without cleavage. The ligand-binding region derived from the GM-CSF receptor α chain or β chain can reduce the activation of monocytes and/or macrophages by GM-CSF through capturing of GM-CSF, thus suppressing the secretion of cytokines such as IL-6 from the monocytes or macrophages.

The chimeric cytokine receptor of the present invention can be used regardless of the type of co-introduced T-cell receptor or CAR, therefore making it widely applicable to adoptive immunotherapy.

### 2. Nucleic Acid Encoding Chimeric Cytokine Receptor

### 2-1. Summary

A second aspect of the present invention is a nucleic acid encoding a chimeric cytokine receptor.

### 2-2. Configuration

The "nucleic acid encoding a chimeric cytokine receptor" may be a nucleic acid encoding any of the chimeric cytokine receptors described in the first aspect. The base sequence of such a nucleic acid is not limited, and examples thereof include a nucleic acid comprising a nucleic acid encoding a ligand-binding region at the 5'-terminal side and an in-frame nucleic acid encoding a T-cell activating region at the 3'-terminal side. Also examples of the base sequence include a codon-optimized base sequence and a base sequence in which an initiation codon (ATG) is added at the 5'-terminal.

In one embodiment, the nucleic acid of the present aspect is a nucleic acid encoding a chimeric cytokine receptor in which the ligand-binding region is derived from an IL-6 receptor, and the T-cell activating region is derived from an IL-7 receptor α chain, an example of which includes a nucleic acid encoding the chimeric cytokine receptor consisting of the amino acid sequence of SEQ ID NO: 24, such as a nucleic acid consisting of the base sequence of SEQ ID NO: 29.

In one embodiment, the nucleic acid of the present aspect is a nucleic acid encoding a chimeric cytokine receptor in which the ligand-binding region is derived from an IL-6 receptor, the T-cell activating region is derived from an IL-7 receptor α chain, and which further comprises a ligand-binding region of gp130 at the N-terminal side of the ligand-binding region derived from the IL-6 receptor, an example of which includes a nucleic acid encoding the chimeric cytokine receptor consisting of the amino acid sequence of SEQ ID NO: 25, such as a nucleic acid consisting of the base sequence of SEQ ID NO: 30.

In one embodiment, the nucleic acid of the present aspect is a nucleic acid encoding a chimeric cytokine receptor in which the ligand-binding region is derived from the IL-1 receptor type 2, and the T-cell activating region is derived from the IL-7 receptor α chain, an example of which includes a nucleic acid encoding the chimeric cytokine receptor consisting of the amino acid sequence of SEQ ID NO: 26, such as a nucleic acid consisting of the base sequence of SEQ ID NO: 31.

In one embodiment, the nucleic acid of the present aspect is a nucleic acid encoding a chimeric cytokine receptor in which the ligand-binding region is derived from the GM-CSF receptor α chain, and the T-cell activating region is derived from the IL-7 receptor α chain, an example of which includes a nucleic acid encoding the chimeric cytokine receptor consisting of the amino acid sequence of SEQ ID NO: 27, such as a nucleic acid consisting of the base sequence of SEQ ID NO: 32.

In one embodiment, the nucleic acid of the present aspect is a nucleic acid encoding a chimeric cytokine receptor in which the ligand-binding region is derived from the GM-CSF receptor β chain, and the T-cell activating region is derived from the IL-7 receptor α chain, an example of which includes a nucleic acid encoding the chimeric cytokine receptor consisting of the amino acid sequence of SEQ ID NO: 28, such as a nucleic acid consisting of the base sequence of SEQ ID NO: 33.

### 3. Gene expression vector comprising nucleic acid encoding chimeric cytokine receptor

### 3-1. Summary

A third aspect of the present invention is a gene vector comprising a nucleic acid encoding a chimeric cytokine receptor in a state which allows for the expression thereof (hereinafter referred to as a "chimeric cytokine receptor expression vector").

### 3-2. Configuration

The gene expression vector of the present aspect is a gene expression vector capable of expressing a chimeric cytokine receptor in a cell, the vector comprising the nucleic acid described in the second aspect and a promoter. In addition to the nucleic acid and the promoter as the above-described components, the gene expression vector may contain components such as a marker gene (selection marker), an enhancer, a terminator, an origin of replication, and a poly A signal, if necessary. Note that the gene expression vector of the present aspect may include a nucleic acid encoding one chimeric cytokine receptor or a combination of nucleic acids encoding two or more chimeric cytokine receptors as the nucleic acid described in the second aspect. When two or more nucleic acids encoding a chimeric cytokine receptor are included, the combination may be any combination, and examples thereof include a combination of a chimeric cytokine receptor comprising the cytokine-binding region of the GM-CSF receptor α chain with a chimeric cytokine receptor comprising the cytokine-binding region of the GM-CSF receptor β chain. The nucleic acid encoding two or more chimeric cytokine receptors may be contained within the same gene expression vector.

As used herein, the term "gene expression vector" refers to a vector containing a gene or gene fragment (hereinafter referred to as a "gene or the like") in a state which allows for the expression thereof, and encompassing an expression unit capable of controlling the expression of the gene or the like. The gene expression vector may be a plasmid vector or a viral vector.

As used herein, the term "a state which allows for the expression" refers to a state in which a gene or the like to be expressed is placed in a region downstream of a promoter under the control of the promoter. As the vector, a plasmid vector, a viral vector and the like are known, and any vector can be used. Usually, the vector may be a plasmid vector that can be easily genetically engineered or a viral vector which can easily introduce a gene into an immune cell.

The plasmid vector can be a commercially available expression vector for mammalian cells, such as a pCI vector or a pSI vector from Promega K.K., or a shuttle vector that can replicate between mammalian cells and bacteria such as *Escherichia coli.*

Examples of the viral vector, which can be used, include a retroviral vector (including a oncoretroviral vector, a lentiviral vector, and a pseudotyped vector), an adenoviral vector, an adeno-associated viral (AAV) vector, a simian viral vector, a vaccinia viral vectors, a Sendai viral vector, an Epstein-Barr viral (EBV) vector, and an HSV vector. A viral vector lacking the ability to replicate may be used to prevent self-replication in an infected cell.

In the case of using a retroviral vector, a suitable packaging cell and a packaging signal sequence may be selected based on the LTR sequence to produce retroviral particles. Examples of the packaging cell include PG13 (ATCC^{®} CRL-10686^{™}), PA317 (ATCC^{®} CRL-9078^{™}), GP+E-86 and GP+envAm-12 (U.S. Patent No. 5,278,056), and Psi-Crip (Proceedings of the National Academy of Sciences of the United States of America, vol. 85, pp. 6460-6464 (1988)). The retroviral particles can also be produced using 293 cells or 293T cells having high transfection efficiencies. Many types of viral vectors produced using packaging cells that can be used for packaging retroviruses and retroviral vectors are commercially available from many companies.

As used herein, the term "promoter" refers to a gene expression regulatory region capable of controlling the expression of a gene or the like located downstream (3'-terminal side) in a cell into which a gene expression vector is introduced. Promoters can be classified into a ubiquitous promoter (systemic promoter) and a site-specific promoter based on the place where a gene or the like under expression control is expressed. The ubiquitous promoter is a promoter that controls the expression of a gene or the like of interest in all cells, that is, in the entire host individual. The site-specific promoter is a promoter that controls the expression of a gene or the like of interest only in specific cells or tissues. The promoter contained in the gene expression vector of the present invention may be either a ubiquitous promoter or a site-specific promoter but is preferably a promoter capable of inducing expression in immune cells.

In addition, promoters are classified into a constitutively active promoter, an expression inducible promoter, or a stage-specific active promoter based on the phase of expression. The constitutively active promoter can constitutively express a gene or the like of interest in a cell. The expression inducible promoter can induce the expression of a gene or the like of interest in a cell at any phase. In addition, the stage-specific active promoter can induce the expression of a gene or the like of interest in a cell only at a specific stage of development. All of the promoters can lead to overexpression of a gene of interest in a host cell and thus can be understood as an overexpression promoter. The promoter contained in the gene expression vector of the present invention is preferably a constitutively active promoter which allows for a long-lasting therapeutic efficacy.

The promoter in the gene expression vector of the present aspect is a promoter that can induce the expression of a nucleic acid encoding a chimeric cytokine receptor in a cell, such as an immune cell. Since the target cell into which the gene expression vector of the present invention is introduced is, in principle, a mammalian cell, particularly a cell of human, for example, an immune cell of human, any promoter capable of expressing a downstream gene in such cells may be used. Examples thereof include a CMV promoter (CMV-IE promoter), an SV40 early promoter, an RSV promoter, an EF1α promoter, an Ub promoter, and a 5' LTR promoter. In the case of a retroviral vector, a nucleic acid encoding a chimeric cytokine receptor can be placed downstream of the 5' LTR promoter to induce its gene expression.

As used herein, the "marker gene" is a gene encoding a marker protein, also referred to as a selection marker or reporter protein. The term "marker protein" refers to a peptide that can determine the presence or absence of expression of a marker gene based on an activity thereof. The activity may be detected directly by the activity itself of the marker protein or indirectly through a metabolite generated by the activity of the marker protein such as a pigment. The detection may be any of biological detection (including detection by binding of a peptide or a nucleic acid such as an antibody and an aptamer), chemical detection (including enzymatic detection), physical detection (including behavioral analytical detection), or sensory detection (including detection by sight, touch, smell, hearing, and taste) of a detector.

The type of the marker protein encoded by the marker gene is not particularly limited as long as the activity can be detected by a method known in the art. Preferred is a marker protein having low invasiveness to a transformant upon detection. Examples thereof include a tag peptide, a drug resistance protein, a chromoprotein, a fluorescent protein, and a luminescent protein.

The "drug resistance protein" is a protein, mostly an enzyme, that imparts resistance to a drug such as an antibiotic added to a medium or the like to a cell, and examples thereof include β-lactamase, which imparts resistance to ampicillin; aminoglycoside 3'-phosphotransferase, which imparts resistance to kanamycin; tetracycline efflux transporter, which imparts resistance to tetracycline; and CAT (chloramphenicol acetyltransferase), which imparts resistance to chloramphenicol.

The "chromoprotein" is a protein, usually an enzyme, that is involved in biosynthesis of a pigment or that allows chemical detection of a transformant by a pigment upon substrate addition. The term "pigment" herein refers to any type of a low molecular weight compound or peptide that can impart a pigment to a transformant. Examples thereof include β-galactosidase (LacZ), β-glucuronitase (GUS), a melanin-based pigment synthesis protein, an ommochrome-based pigment, and a pteridine-based pigment.

The term "fluorescent protein" refers to a protein that emits fluorescence of a specific wavelength when irradiated with excitation light of a particular wavelength. The fluorescent protein may be either natural or non-natural, and the excitation wavelength and fluorescence wavelength are not particularly limited. Specific examples thereof include CFP, RFP, DsRed (including a derivative such as 3xP3-DsRed), YFP, PE, PerCP, APC, and GFP (including a derivative such as EGFP and 3xP3-EGFP).

The "luminescent protein" refers to a substrate protein that can emit light without the need for excitation light or an enzyme that catalyzes the emission of light from the substrate protein. Examples thereof include luciferin or aequorin as the substrate protein and luciferase as the enzyme.

As used herein, the "enhancer" is not particularly limited as long as it can enhance the expression efficiency of a gene or a fragment thereof in a vector.

As used herein, the "terminator" is a sequence capable of terminating transcription of an expressed gene or the like by the activity of the above-described promoter. The type of the terminator is not particularly limited. The terminator is preferably derived from the same species as the promoter and particularly preferably paired with the above-described promoter on the genome in a single gene expression control system.

### 4. Host cell containing gene expression vector

### 4-1. Summary

A fourth aspect of the present invention is a host cell. The host cell of the present aspect contains a gene vector comprising a nucleic acid encoding a chimeric cytokine receptor in a state which allows for the expression thereof. The host cell of the present aspect is, for example, a CAR-T cell containing a chimeric antigen receptor (CAR) expression vector.

### 4-2. Configuration

The host cell of the present aspect contains a chimeric cytokine receptor expression vector as an essential component and a CAR expression vector and/or an IL-1 receptor type 2 expression vector as an optional component.

The chimeric cytokine receptor expression vector as an essential component is in accordance with the description of the third aspect. The host cell of the present aspect may include one chimeric cytokine receptor expression vector or a combination of two or more chimeric cytokine receptor expression vectors. When two or more chimeric cytokine receptor expression vectors are included, the combination may be any combination, and examples thereof include a combination of an expression vector expressing a chimeric cytokine receptor comprising the cytokine-binding region of the GM-CSF receptor α chain and an expression vector expressing a chimeric cytokine receptor comprising the cytokine-binding region of the GM-CSF receptor β chain.

The type of the host cell of the present aspect is not limited. Examples of host cells include immune cells, peripheral blood mononuclear cells (PBMCs), umbilical cord blood mononuclear cells, skin keratinocytes, mesenchymal stem cells, hematopoietic stem cells, various cancer cell lines, and neural stem cells, iPS cells, and ES cells. A preferred host cell is an immune cell. The immune cell may be a T cell, an NK cell, or a macrophage. The T cell is preferably a naive T cell and a memory T cell. The host cell such as an immune cell may be derived from a living organism, an immortalized cell line, or a cell differentiated from an ES cell or an induced pluripotent stem cell (iPS cell).

As used herein, the term "chimeric antigen receptor (CAR) expression vector" refers to a gene expression vector containing a nucleic acid encoding a chimeric antigen receptor (CAR) and a promoter and capable of expressing the CAR in a cell. In addition to the nucleic acid and the promoter as the above-described components, the CAR expression vector may contain components such as a marker gene (selection marker), an enhancer, a terminator, an origin of replication, and a poly A signal, if necessary. The CAR expression vector may be a plasmid vector or a viral vector.

The type of a CAR expressed by a CAR expression vector is not limited, nor is the type of an antigen targeted by the CAR. Examples of the antigen include a viral antigen, a bacterial antigen, a parasite antigen, a cell surface marker on a target cell associated with a certain pathology, a tumor antigen, and a surface molecule of an immune cell. The CAR preferably targets an antigen on the tumor cell surface.

The term "IL-1 receptor type 2 expression vector" refers to a gene expression vector containing a nucleic acid encoding an IL-1 receptor type 2 and a promoter and capable of expressing an IL-1 receptor type 2 in a cell. In addition to the nucleic acid and the promoter as the above-described components, the IL-1 receptor type 2 expression vector may contain components such as a marker gene (selection marker), an enhancer, a terminator, an origin of replication, and a poly A signal, if necessary. The IL-1 receptor type 2 expression vector may be a plasmid vector or a viral vector.

### 4-3. Effect

Based on the ligand-binding region at the N-terminal side of the chimeric cytokine receptor, the host cell of the present invention can capture extracellular cytokines around the host cell and can optionally absorb the cytokine into the cell. Furthermore, based on the T-cell activating region, the host cell has enhanced proliferative ability and cytotoxic activity, and therapeutic efficacy based on the cytotoxic activity can be sustained in the long term.

The host cell of the present aspect containing the CAR expression vector can be used as a CAR-T cell for CAR-T cell therapy.

When the host cell of the present aspect contains a gene expression vector encoding a chimeric cytokine receptor whose ligand-binding region is derived from an IL-6 receptor, and an IL-1 receptor type 2 expression vector, the host cell of the present aspect can capture both IL-6 and IL-1β, thus suppressing both cytokine release syndrome and neurotoxicity syndrome.

### 5. Cell preparation

### 5-1. Summary

A fifth aspect of the present invention is a cell preparation. The cell preparation of the present aspect contains the host cell of the fourth aspect as an active ingredient and can be used for adoptive immunotherapy. According to the cell preparation of the present aspect, cytokine release syndrome and neurotoxicity syndrome can be suppressed in adoptive immunotherapy, and therapeutic efficacy can be maintained for a long term.

### 5-2. Definition

As used herein, the term "subject" refers to a subject to which the cell preparation of the present aspect is applied, such as a tissue, an organ, or an individual. In the case of an individual, for example, the subject is a mammal, preferably a human individual. The human individual may be a patient, such as a cancer patient.

As used herein, the term "information of the subject" refers to a variety of information on the characteristics and conditions of the subject, such as age, weight, sex, general health, presence of disease, progression and severity of disease, drug sensitivity, presence of concomitant drugs, and resistance to treatment when the subject is a human individual.

As used herein, the term "treatment" refers to alleviation or elimination of symptoms associated with a disease and/or prevention or suppression of disease progression, as well as cure of the disease.

As used herein, "disease" is not limited, and examples of the disease include cancer, an inflammatory disease, an autoimmune disease, hepatitis, and an infectious disease. Examples of the infectious disease include a viral infection, such as influenza and HIV, a bacterial infection, and a fungal infection. The disease is preferably cancer.

As used herein, the type of "cancer" is not limited, and examples thereof include adenocarcinoma, squamous cell carcinoma, small cell carcinoma, and large cell carcinoma. Specific examples of the type of cancer include malignant melanoma, oral cavity cancer, larynx cancer, pharyngeal cancer, thyroid cancer, lung cancer, breast cancer, esophageal cancer, stomach cancer, colorectal cancer (including colon cancer and rectal cancer), small intestine cancer, bladder cancer, prostate cancer, testicular cancer, endometrial cancer, cervical cancer, endometrial cancer, ovarian cancer, stomach cancer, renal cancer, liver cancer, pancreatic cancer, biliary tract cancer (including gallbladder cancer and bile duct cancer), brain tumor, head and neck cancer, mesothelioma, osteosarcoma, soft tissue sarcoma, glioma, neuroblastoma and other pediatric tumors, blood cancer, lymphoma, and myeloma. Examples of the blood cancer include leukemia (e.g., B-cell leukemia, acute lymphoblastic leukemia, chronic lymphocytic leukemia), lymphoma (e.g., non-Hodgkin's lymphoma), and myeloma (e.g., multiple myeloma). In the present specification, cancer may be either solid cancer or blood cancer.

### 5-3. Configuration

### 5-3-1. Component

Components of the cell preparation of the present aspect is described below. The cell preparation of the present aspect contains one or more active ingredients, a solvent, and/or a carrier as essential components. Each component is described in detail below.

### (Active ingredient)

The cell preparation of the present aspect encompasses the host cell of the fourth aspect as an essential active ingredient. The cell preparation of the present aspect may contain one or more types of host cells.

The content of the active ingredient contained in the cell preparation of the present invention is not particularly limited. Generally, the content varies depending on the type of the active ingredient, the dosage form, and the type of a solvent or a carrier that is described below as other components. Therefore, the content may be determined as appropriate in consideration of each condition. It is sufficient that an effective amount of the active ingredient is contained in a single dose of the cell preparation. However, when it is necessary to administer a large dose of the cell preparation to a subject in order to obtain pharmacological effects of the active ingredient, the cell preparation may be administered in several divided doses to reduce the burden on the subject. In this case, the amount of the active ingredient should be an effective amount in total.

The term "effective amount" refers to an amount that is necessary for exhibiting functions as an active ingredient and imparts little or no adverse side effects to a subject to which the active ingredient is applied. This effective amount may vary depending on various conditions such as the information of the subject, an application route, and the number of applications. Thus, when the cell preparation of the present aspect is used as a medicine, the content of the active ingredient is ultimately determined by the judgment of, for example, a doctor or a pharmacist.

The amount of host cells contained in the cell preparation of the present aspect is, for example, 10⁵ cells to 10¹⁰ cells or 10⁶ cells to 10⁹ cells, preferably 10⁷ cells to 10⁸ cells. Examples thereof include, but are not limited to, 0.2 to 5.0 × 10⁶ cells/kg for children weighing 50 kg or less, 0.1 to 2.5 × 10⁸ cells for children weighing over 50 kg, and 0.6 to 6.0 × 10⁸ cells for adults.

### (Solvent)

The cell preparation of the present invention can contain a pharmaceutically acceptable solvent, if necessary. The term "pharmaceutically acceptable solvent" refers to a solvent commonly used in the field of formulation technology. Examples thereof include water or an aqueous solution and an organic solvent. Examples of the aqueous solution include an isotonic solution containing saline, glucose, or the other additives, a phosphate buffer, a phosphate buffered saline, a sodium acetate buffer, a solution of glycol or ethanol. Examples of the additive include D-sorbitol, D-mannose, D-mannitol, sodium chloride, a low concentration nonionic surfactant, and a polyoxyethylene sorbitan fatty acid esters. The organic solvent includes ethanol.

### (Carrier)

The cell preparation of the present invention can contain a pharmaceutically acceptable carrier, if necessary. The term "pharmaceutically acceptable carrier" refers to an additive commonly used in the field of formulation technology. Examples thereof include an excipient and human serum albumin.

Examples of the excipient include sugars such as a monosaccharide, a disaccharide, a cyclodextrin, and a polysaccharide; an inorganic acid salt such as a hydrochloride, a hydrobromide, a phosphate, and a sulfate; a salt of an organic acid such as an acetate, a propionate, a malonate, and a benzoate; a metal salt; citric acid; tartaric acid; glycine; polyethylene glycol; kaolin; and silicic acid, or a combination thereof.

In addition to the above, the cell preparation may also contain, as appropriate and if necessary, a solubilizer, a suspension, a diluent, a dispersant, a surfactant, an analgesic agent, a stabilizer, an absorption enhancer, an extender, a preservative, an antiseptic, an antioxidant, a buffer, an isotonic agent, and another agent, which are usually used for example, in a pharmaceutical composition.

The carrier is used to avoid or suppress degradation of the above-described active ingredient, for example, by an enzyme in the body of a subject, to facilitate formulation and administration method, and to maintain the dosage form and drug efficacy, and may be used as appropriate and if necessary.

### 5-3-2. Dosage form

The dosage form of the cell preparation of the present invention is not particularly limited as long as the active ingredient can be delivered to a target site without inactivation in the body of a subject.

The specific dosage form varies according to application methods described below. The application methods can be broadly classified into parenteral administration and oral administration, and preferred is parenteral administration.

If the administration method is parenteral, the preferred dosage form is a liquid agent that can be administered directly to the target site or systemically administered via the circulatory system. Good examples of the liquid agent include an injection agent, which can be formulated by appropriately mixing with, for example, an excipient, a suspension, a surfactant, a stabilizer, a pH adjusting agent, and another agent, in addition to a solvent, in a unit dose form required for generally accepted pharmaceutical practice.

### 5-3-3. Application method

The application method of the cell preparation of the present invention is not particularly limited, nor is the route of administration. For example, the cell preparation may be administered parenterally. The parenteral administration can be further subdivided into systemic and local administration. The local administration includes, for example, intradermal administration, intramuscular administration, subcutaneous administration, intraperitoneal administration, intranasal administration, intratumoral administration, tissue administration, and organ administration, while the systemic administration in the parenteral administration method includes intracirculatory administration, for example, intravenous administration (IV injection), intraarterial administration, and intralymphatic administration. A preferred route of administration is intravenous, which may be an infusion administered by drip (e.g., a single intravenous infusion).

### 5-4. Effect

According to the cell preparation of the present aspect, there is provided a method of treating and/or preventing a disease, the method comprising administering the cell preparation of the present invention to a subject. The disease may be cancer, for example, and the subject may be a cancer patient. The method of treatment/prevention is characterized by long-lasting effects of *e.g.,* cytotoxic activity and reduced side effects of *e.g.,* cytokine release syndrome and neurotoxicity syndrome. This method of treatment/prevention may be adoptive immunotherapy, such as CAR-T cell therapy.

According to the cell preparation of the present aspect, there is also provided a method of providing antitumor immunity to a subject, the method comprising administering the cell preparation to a subject.

Also provided is a use of the chimeric cytokine receptor, gene expression vector, host cell, or cell preparation of the present invention in the manufacture of a medicine for treating and/or preventing a disease, such as cancer.

### 6. Method of producing chimeric antigen receptor (CAR)-transduced cell having long-lasing cytotoxic activity

### 6-1. Summary

A sixth aspect of the present invention is a method of producing a chimeric antigen receptor (CAR)-transduced cell having a long-lasting cytotoxic activity. The production method of the present aspect comprises a peripheral blood mononuclear cell isolation step and a vector introduction step as essential steps. According to the production method of the present aspect, a CAR-transduced cell having a long-lasting cytotoxic activity can be produced. The CAR-transduced cell produced by the production method of the present aspect can be used for CAR-T cell therapy whose therapeutic efficacy is maintained in the long term.

As used herein, the term "long-lasting cytotoxic activity" means that the cytotoxic activity against a target cell, such as a cancer cell, lasts for a long time, and "long-lasting" means, for example, that the cytotoxic activity lasts longer than the duration of the therapeutic efficacy by a therapeutic cell in conventional adoptive immunotherapy. More specifically, the term "long-lasting" means that the duration of the therapeutic efficacy lasts for a longer time compared to a CAR-transduced cell into which the chimeric cytokine receptor expression vector of the present invention has not been introduced. For example, detectable cytotoxic activity lasts for 2 days or more, 3 days or more, 4 days or more, 5 days or more, 6 days or more, 1 week or more, 10 days or more, 2 weeks or more, 3 weeks or more, 24 days or more, 4 weeks or more, 1 month or more, 5 weeks or more, 38 days or more, 40 days or more, 6 weeks or more, 45 days or more, 7 weeks or more, 50 days or more, 55 days or more, 60 days or more, 2 months or more, 3 months or more, 4 months or more, 5 months or more, 6 months or more, or 1 year or more. Note that the term "lasting" cytotoxic activity means that at least detectable cytotoxic activity may be maintained over any of the above periods, and for example, a higher cytotoxic activity may be maintained as compared with a CAR-transduced cell into which the chimeric cytokine receptor expression vector of the present invention has not been introduced.

### 6-2. Method

The method of producing a CAR-transduced cell of the present aspect comprises a peripheral blood mononuclear cell isolation step and a vector introduction step as essential steps and an expansion culture step as an optional step. Each step is described in detail below.

### (Peripheral blood mononuclear cell isolation step)

The "peripheral blood mononuclear cell isolation step" refers to a step of isolating peripheral blood mononuclear cells from the peripheral blood of a subject. This step is intended to isolate peripheral blood mononuclear cells containing immune cells.

As used herein, the term "peripheral blood mononuclear cell (PBMC)" means a cell or cell population containing monocytes and/or lymphocytes such as T cells, which have been separated from the peripheral blood of a human or animal.

The method of separating peripheral blood mononuclear cells is not limited. For example, peripheral blood mononuclear cells can be separated by density gradient centrifugation or hemolysis. Density gradient centrifugation can be performed by centrifuging a diluted whole blood sample which is layered on a solvent. Ficoll-hypaque^{®} or the like can be used as the solvent, an example of which includes a solvent adjusted to a density of 1.077 g/mL. As a result of the density gradient centrifugation, the mononuclear cell component can be separated from red blood cells, granulocytes, and plasma as an intermediate layer. Hemolysis is a method of removing red blood cells with a hypoosmotic solution, but the granulocyte component is not removed in this method. Thus, this isolating step can be usually performed by density gradient centrifugation.

### (Vector introduction step)

The "vector introduction step" is a step of introducing the gene expression vector containing a nucleic acid encoding a chimeric cytokine receptor described in the third aspect (hereinafter referred to as a "chimeric cytokine receptor expression vector") and a CAR expression vector to the peripheral blood mononuclear cells isolated in the peripheral blood mononuclear cell isolation step. The CAR expression vector is a gene expression vector containing a nucleic acid encoding a chimeric antigen receptor (CAR) in a state which allows for the expression thereof, and the specific configuration thereof is in accordance with the description of the fourth aspect. Cells into which a chimeric cytokine receptor expression vector and a CAR expression vector have been introduced in this step can be used, for example, as CAR-T cells for adoptive immunotherapy such as CAR-T cell therapy.

The method of introducing each vector of the chimeric cytokine receptor expression vector and the CAR expression vector into peripheral blood mononuclear cells is not particularly limited.

When each vector is a viral vector, methods of viral infection of cells are known in the art, and the viral vector may be introduced using a functional substance that improves viral infection efficiency, such as a fibronectin or a fibronectin fragment (*e.g*., RetroNectin^{®} or Vecofusin-1^{®}, a fibronectin fragment with a heparin binding site).

The viral infection methods using RetroNectin include the following: after a cell culture plate is treated with RetroNectin, the bottom surface of the plate is blocked with a 2% BSA/PBS solution for 30 minutes, followed by washing with PBS. Subsequently, a retroviral solution derived from PG13 packaging cells is loaded onto the plate, and the culture plate is centrifuged at 32°C and 2,000 g for two hours. After centrifugation, the viral solution is removed and the cells are seeded on the plate.

When each vector is a non-viral vector such as a plasmid, a gene introduction method (transformation method) known in the art described, for example, in Green & Sambrook, 2012, Molecular Cloning: A Laboratory Manual Fourth Ed., Cold Spring Harbor Laboratory Press, Cold Spring Harbor, New York may be used, and examples thereof include a lipofection method, an electroporation method, a microinjection method, a calcium phosphate method, a DEAE-Dextran method, and particle bombardment.

The peripheral blood mononuclear cells into which the vector has been introduced in this step can usually be directly used, but, if necessary, only the cells into which the vector has been introduced may be separated.

### (Expansion culture step)

The "expansion culture step" is a step of stimulating peripheral blood mononuclear cells obtained after the peripheral blood mononuclear cell isolation step with an anti-CD3 antibody, which can be performed as an optional step between the peripheral blood mononuclear cell isolation step and the vector introduction step described above. The anti-CD3 antibody used in this step is not limited as long as it can stimulate peripheral blood mononuclear cells, and for example, a soluble anti-CD3 antibody or a cell expressing a membrane-bound anti-CD3 antibody may be used.

In this step, peripheral blood mononuclear cells stimulated with an anti-CD3 antibody may also be cultured in the presence of cytokines such as IL-2, thereby maintaining the proliferation of the peripheral blood mononuclear cells.

The expansion culture step can improve the efficiency of T cell proliferation and CAR introduction.

### Examples

### <Example 1: Production of chimeric cytokine receptors IL6R-ca7R and GP130-IL6R-ca7R>

### (Object)

A chimeric cytokine receptor IL6R-ca7R which comprises a portion of the interleukin-6 receptor α subunit (hereinafter referred to as "IL6RA") and a portion of the interleukin-7 receptor α subunit (hereinafter referred to as "IL7RA") is produced. In addition, a chimeric cytokine receptor GP130-IL6R-ca7R which comprises a portion of Glycoprotein 130 (hereinafter referred to as "gp130"), a portion of IL6RA, and a portion of IL7RA is produced.

### (Method and results)

### (1) Production of chimeric cytokine receptor IL6R-ca7R

IL6R-ca7R was produced as an artificial receptor in which a signal peptide, a ligand-binding region, and a T-cell activating region were linked in this order from the N-terminal side (Figure 2A). Each component is as described below.

The signal peptide used was a silkworm Fibroin L protein-derived signal peptide (SEQ ID NO: 34).

The ligand-binding region consists of the extracellular domain of IL6RA, specifically, positions 20 to 365 in full-length human IL6RA of SEQ ID NO: 11.

The T-cell activating region consists of a portion from the hinge region to the intracellular domain in IL7RA having a constitutively active mutation, and specifically consists of the amino acid sequence in which constitutively active insertion mutation Pro-Pro-Cys-Leu (SEQ ID NO: 2) is inserted between position 243 and position 244 in positions 232 to 459 of the full-length human IL7RA of SEQ ID NO: 1.

The full-length amino acid sequence of IL6R-ca7R is shown in SEQ ID NO: 24, and the base sequence of a gene encoding IL6R-ca7R (hereinafter referred to as an "IL6R-ca7R gene") is shown in SEQ ID NO: 29.

### (2) Production of chimeric cytokine receptor GP130-IL6R-ca7R

GP130-IL6R-ca7R was produced as an artificial receptor in which a gp130-derived ligand-binding region, a linker peptide, an IL6RA-derived ligand-binding region, and a T-cell activating region were linked in this order from the N-terminal side (Figure 2B). Each component is as described below.

The gp130-derived ligand-binding region consists of the extracellular domain of gp130, which contains a gp130-derived signal peptide at the N-terminal thereof, and specifically consists of positions 1 to 326 in the full-length human gp130 of SEQ ID NO: 13.

The IL6RA-derived ligand-binding region consists of the extracellular domain ofIL6RA, and specifically consists of positions 110 to 365 in the full-length human IL6RA of SEQ ID NO: 11.

The T-cell activating region consists of the amino acid sequence in which constitutively active insertion mutation Pro-Pro-Cys-Leu (SEQ ID NO: 2) is inserted between position 243 and position 244 in positions 232 to 459 of the full-length human IL7RA of SEQ ID NO: 1 as in above (1).

The full-length amino acid sequence of GP130-IL6R-ca7R is shown in SEQ ID NO: 25, and the base sequence of a gene encoding GP130-IL6R-ca7R (hereinafter referred to as a "GP130-IL6R-ca7R gene") is shown in SEQ ID NO: 30.

### <Example 2: Cell surface expression of chimeric cytokine receptor>

### (Object)

Chimeric cytokine receptors IL6R-ca7R and GP130-IL6R-ca7R are introduced into T cells together with a chimeric antigen receptor (CAR) and their cell surface expression is analyzed by flow cytometry.

### (Method and results)

A medium for T cells used was a RPMI 1640 medium supplemented with 10% FBS, penicillin (100 units/mL), streptomycin (100 µg/mL), and a recombinant IL2 (100 IU/mL).

On the first day of culture, peripheral blood mononuclear cells (HHU20180703 or HHU20180821) were stimulated (primed) with an anti-CD3 antibody. For priming, a K562 cell line, in which a single-chain variable fragment (scFV) derived from an anti-CD3 antibody (clone OKT3) and co-stimulatory molecule CD80 were expressed on the cell surface, was used. The K562 cell line was treated with mitomycin to stop cell division, and then the K562 cell line was co-cultured with peripheral blood mononuclear cells in a ratio of 1:7. Note that the priming was performed in all of the following Examples to improve T cell proliferation and CAR introduction efficiency.

On day 2 of culture, the IL6R-ca7R gene or GP130-IL6R-ca7R gene produced in Example 1 was introduced into T cells together with a FMC63-28z CAR gene with retrovirus. Specifically, the pMX plasmid (Kitamura T. et al., Exp Hematol., 2003, 31:1007-1014.) as a retroviral plasmid was introduced into Plat-E packaging cells by transient transfection using TranslT-293 (Mirus Bio), and the resulting ecotropic retroviral vector was stably introduced into PG13 packaging cells. The resulting PG13 cell-derived viral vector was introduced into T cells using RetroNectin (Takara Bio Inc.). Note that the FMC63-28z CAR, in which a single-chain variable fragment derived from an anti-CD19 antibody (clone FMC63; Nicholson et al., Mol Immunol. 1997, 34 (16-17): 1157-65.), the transmembrane region and cytoplasmic domain of CD28, and the cytoplasmic domain of CD3z were linked, was produced with reference to the literature (Kochenderfer J.N., et al., J Immunother. 2009 Sep;32(7):689-702.).

Next, on day 5 of culture, T cells in the CD8-positive fraction were labeled with anti-IL6RA antibodies (from BioLegend, Inc., 352812) and analyzed by flow cytometry for cell surface expression of chimeric cytokine receptors and/or IL6RA. Specifically, after the cells were stained with the antibodies, data were acquired by BD LSRFortessa (BD Biosciences) and analyzed using Flowjo software (BD Biosciences).

A representative flow cytometry plot is shown in Figure 3. As shown in Figure 3, 90% or more and 80% or more of CAR-T cells transduced with the chimeric cytokine receptors IL6R-ca7R or GP130-IL6R-ca7R, respectively, were positive, whereas few positive cells were detected in control CAR-T cells to which only FMC63-28z CAR gene was introduced.

### <Example 3: Kinetics of chimeric cytokine receptor internalization with IL-6 administration>

### (Object)

Recombinant IL-6 (interleukin-6) is administered to CAR-T cells in which a chimeric cytokine receptor is co-transduced and the kinetics of chimeric cytokine receptor internalization is analyzed.

### (Method and results)

The GP130-IL6R-ca7R gene was introduced into T cells with a NGFR-FMC63-28z CAR gene (hereinafter, referred to as "GP130-IL6R-ca7R-transduced CAR-T cells") with retrovirus in the same manner as in Example 2. The NGFR-FMC63-28z CAR means a tNGFR-Furin-SGSG-P2A-FMC63-28z CAR, which is a polypeptide containing, in this order from the N-terminal side, truncated NGFR as a labeling marker, a Furin protease cleavage sequence (RAKR), a flexible linker (SGSG), a porcine teschovirus virus-derived P2A sequence, and a FMC63-28z CAR.

Recombinant IL-6 (from PeproTech, Inc., 200-06) was added at 50 ng/mL to GP130-IL6R-ca7R-transduced CAR-T cells. After the cells were allowed to stand in the presence of IL-6 for 30 minutes, the medium was replaced with an IL-6 free medium to remove IL-6, and the cells were cultured for another 3.5 hours.

T cells in the CD8-positive fraction before the addition of IL-6 (0 min), after 30 minutes of standing in the presence of IL-6 (30 min), and after 3.5 hours of culturing without IL-6 (4 hour) were analyzed by flow cytometry by labeling chimeric cytokine receptors on the cell surface with anti-IL6RA antibodies. In this Example, T cells transduced with only the NGFR-FMC63-28z CAR gene were used as control cells.

The results of flow cytometry analysis are shown in Figure 4. In the GP130-IL6R-ca7R-transduced CAR-T cells, chimeric cytokine receptors were internalized inside the cells in the presence of IL-6 (Figure 4, 30 min) and were recycled to the cell surface after IL-6 was removed (Figure 4, 4 h). In contrast, such kinetics of receptor internalization was not observed in the control cells.

The quantification results of flow cytometry are shown in Figure 5. In the GP130-IL6R-ca7R-transduced CAR-T cells, chimeric cytokine receptors on the cell surface were reduced in the presence of IL-6 (Figure 5A, 0 min vs. 30 min) and recovered after IL-6 was removed (Figure 5A, 30 min vs. 4 h). The proportion of the cells labeled with anti-IL6RA antibodies was also reduced in the presence of IL-6 (Figure 5B, 0 min vs. 30 min) and then recovered after IL-6 was removed (Figure 5B, 30 min vs. 4 h). Such receptor internalization kinetics was not observed in the control cells.

These results showed that in the GP130-IL6R-ca7R-transduced CAR-T cells, chimeric cytokine receptors were internalized into the cells by the addition of IL-6.

### <Example 4: IL-6 capturing ability of chimeric cytokine receptor-transduced CAR-T cells>

### (Object)

Recombinant IL-6 is added to CAR-T cells co-transduced with a chimeric cytokine receptor, and the IL-6 concentration in the supernatant is then measured and the IL-6 capturing ability is evaluated.

### (Method and results)

### (1) Analysis of IL-6 capturing ability of chimeric cytokine receptor-transduced CAR-T cells

The FMC63-28z CAR gene encoding a CAR against CD19 and the IL6R-ca7R gene or GP130-IL6R-ca7R gene were co-introduced into T cells using retrovirus. Gene introduction by the retrovirus was performed in the same manner as in Example 2. Hereinafter, T cells transduced with both the CAR gene and the chimeric cytokine receptor gene (IL6R-ca7R gene or GP130-IL6R-ca7R gene) are referred to as "chimeric cytokine receptor-transduced CAR-T cells" ("IL6R-ca7R-transduced CAR-T cells" or "GP130-IL6R-ca7R-transduced CAR-T cells"). The cells on day 5 of culture were plated at 2.5 × 10⁵ cells/well in a 48-well plate, and recombinant IL-6 was added to a final concentration of 3,000 pg/mL. After 48 hours from the addition of IL-6, the culture supernatant was collected to measure IL-6 concentration in the supernatant by ELISA. The experimental procedure is shown in Figure 6A. Note that in this Example, T cells transduced with only the NGFR-FMC63-28z CAR gene were used as control cells.

The results of measuring the IL-6 concentration in the culture supernatant are shown in Figure 6B. The IL-6 concentration in supernatant was greatly reduced in the IL6R-ca7R-transduced CAR-T cells compared to the control cells, and most of the added IL-6 was captured. The IL-6 concentration in the supernatant was further reduced in the GP130-IL6R-ca7R-transduced CAR-T cells, indicating that almost all of the IL-6 was captured.

### (2) Investigation of the involvement of chimeric cytokine receptors on cell membrane surface in the IL-6 capture

Next, it was examined whether the IL-6 capture was by the receptors on the cell membrane surface or by the receptors secreted into the culture supernatant. The IL6R-ca7R-transduced CAR-T cells or GP130-IL6R-ca7R-transduced CAR-T cells were plated at 2.5 × 10⁵ cells/well in a 48-well plate, and after 48 hours, only the culture supernatant was transferred to another 48-well plate, to which recombinant IL-6 was added to a final concentration of 3,000 pg/mL. After 24 hours, the supernatant was collected to measure the IL-6 concentration by ELISA.

The results are shown in Figure 6C. In Figure 6C, the results of measuring the IL-6 concentration in the supernatant after addition of IL-6 to the culture supernatant of the control cells, the IL6R-ca7R-transduced CAR-T cells, and the GP130-IL6R-ca7R-transduced CAR-T cells are shown as "control_sup," "IL6R-ca7R_sup," and "GP130-IL6R-ca7R_sup." In the same manner as in above (1), the results of measuring the IL-6 concentration in the supernatant after addition of IL-6 to the cells are shown as "control_cell," "IL6R-ca7R_cell," and "GP130-IL6R-ca7R_cell." As shown in Figure 6C, no significant effect of capturing IL-6 was detected when IL-6 was added to the culture supernatant of the cells.

The above results indicated that the IL-6 capture effect was due to the chimeric cytokine receptors on the cell membrane surface and not by the chimeric cytokine receptors secreted into the culture supernatant. The IL-6 captured by the chimeric cytokine receptors on the cell membrane surface was considered absorbed into the cells.

### <Example 5: Activation of JAK-STAT pathway based on chimeric cytokine receptor>

### (Object)

Phosphorylated STAT3/STAT5 levels are measured in chimeric cytokine receptor-transduced CAR-T cells for investigating the activation of JAK-STAT pathway.

### (Method and results)

The FMC63-28z CAR gene and the IL6R-ca7R gene or GP130-IL6R-ca7R gene were co-introduced into T cells in the same manner as in Example 4. The chimeric cytokine receptor-transduced CAR-T cells on day 6 from the priming on day 1 of culture were allowed to rest in a cytokine-free medium for one day, and then recombinant IL-6 was added to the medium at 10 ng/mL and allowed to stand for 30 minutes at 37°C. Then, phosphorylated STAT3 (pSTAT3) and phosphorylated STATS (pSTAT5) were detected in the CD3-positive T cell fraction by intracellular flow cytometry.

The results of analyzing pSTAT are shown in Figure 7. Figure 7 shows the distribution of pSTAT3 signal intensity after a 1-day rest of the IL6R-ca7R-transduced CAR-T cells and GP130-IL6R-ca7R-transduced CAR-T cells in the cytokine-free medium (Figure 7A), the distribution of pSTAT3 signal intensity after addition of IL-6 (Figure 7B), and the results of quantifying the fluorescence intensity which indicates pSTAT3 (Figure 7C).

The results of analyzing pSTAT5 are shown in Figure 8. Figure 8 shows the distribution of pSTAT5 signal intensity after a 1-day rest (Figure 8A) and the results of quantifying the fluorescence intensity which indicates pSTAT5 before and after addition of IL-6 after a 1-day rest (Figure 8B).

These results indicated that the JAK-STAT pathway was constitutively active in the IL6R-ca7R-transduced CAR-T cells and the GP130-IL6R-ca7R-transduced CAR-T cells regardless of cytokine addition, and that the STAT3 signal was further activated by the addition of IL-6.

### <Example 6: Proliferation ability of chimeric cytokine receptor-transduced CD19-targeting CAR-T cell>

### (Object)

The cell proliferation ability of chimeric cytokine receptor-transduced CD19-targeting CAR-T cells is evaluated.

### (Method and results)

A CAR gene targeting CD19 as an antigen (FMC63-28z CAR gene or FMC63-BBz CAR gene) and an IL6R-ca7R gene or a GP130-IL6R-ca7R gene were co-introduced into T cells. Hereinafter, T cells transduced with both the CAR gene targeting CD19 as an antigen (FMC63-28z CAR gene or FMC63-BBz CAR gene) and the gene encoding the chimeric cytokine receptor (IL6R-ca7R gene or GP130-IL6R-ca7R gene) are referred to as "chimeric cytokine receptor-transduced CD19-targeting CAR-T cells" ("IL6R-ca7R-transduced CD19-targeting CAR-T cells" or "GP130-IL6R-ca7R-transduced CD19-targeting CAR-T cells"). The FMC63-BBz CAR is a CAR containing a single-chain variable fragment derived from an anti-CD19 antibody (clone FMC63) and a part of 4-lBB, and was produced based on the sequence described in the literature (Milone M.C., et al., Mol Ther, 2009, 17(8):1453-64.). On day 5 of culture, chimeric cytokine receptor-transduced CD19-targeting CAR-T cells and control cells transduced with the CAR gene alone were plated at 2.5 × 10⁵ cells/well in a 48-well plate and co-cultured at 1:1 with the CD19-positive tumor cell line NALM6 (Cell Resource Center for Biomedical Research, Institute of Development, Aging and Cancer, Tohoku University, TKG 0413). The culture was performed in a RPMI 1640 medium containing 10% FBS, penicillin (100 units/mL), and streptomycin (100 µg/mL) under conditions without the addition of cytokines, and the medium was exchanged on day 5 of culture. The cell number of the chimeric cytokine receptor-transduced CD19-targeting CAR-T cells and control cells were counted 7 days after the start of co-culture to evaluate their cell proliferation ability.

The fold change in the number of cells from the start of co-culture in the case of using the FMC63-28z CAR gene as the CAR gene is shown in Figure 9A. The proliferation rate of the control cells in the co-culture was 3 times or less, whereas the proliferation rates of the IL6R-ca7R-transduced CD19-targeting CAR-T cells and the GP130-IL6R-ca7R-transduced CD19-targeting CAR-T cells were both 12 times or higher. Similar results were obtained in the case of using the FMC63-BBz CAR gene as the CAR gene (Figure 9B).

These results indicated that the proliferation ability of the CAR-T cells was greatly enhanced by the introduction of the chimeric cytokine receptor, regardless of the type of CAR.

### <Example 7: Functional evaluation of chimeric cytokine receptor-transduced CD19-targeting CAR-T cells>

### (Object)

Chimeric cytokine receptor-transduced CD19-targeting CAR-T cells are evaluated for their function. Specifically, the proportion of IFN-γ producing cells, the amount of Granzyme B production, and the tumor cell survival rate are examined.

### (Method and Results)

### (1) IFN-y producing cell

The GP130-IL6R-ca7R-transduced CD19-targeting CAR-T cells were co-cultured with the CD19-positive tumor cell line NALM6 at 1:1. Brefeldin A (BioLegend, Inc., 420601) at a 1/1000 dilution was administered 2 hours after the start of co-culture. After another 4 hours, the T cells were stained with an anti-IFN-y antibody (BioLegend, Inc., 502528), and IFN-γ-producing cells in the CD4-positive T cell fraction and CD8-positive T cell fraction were analyzed by flow cytometry.

The results are shown in Figure 10. The proportion of IFN-γ-producing cells was significantly increased in the GP130-IL6R-ca7R-transduced CD19-targeting CAR-T cells compared to the control cells.

### (2) Amount of Granzyme B production

The GP130-IL6R-ca7R-transduced CD19-targeting CAR-T cells and the CD19-positive tumor cells (K562-CD19 cell line or Raji cell line) were co-cultured at 1:1. The K562-CD19 cells used were produced by stably introducing the CD19 gene into the K562 cell line (JCRB Cell Bank, JCRB0019). The Raji cell line used was obtained from the JCRB Cell Bank (JCRB Cell Bank, JCRB9012). After 3 hours from the start of co-culture, the amount of Granzyme B production was analyzed by intracellular flow cytometry using a Granzyme B antibody (Invitrogen, GRB04).

The distribution of the amount of Granzyme B (GZMB) produced after co-culture with the K562-CD19 cell line is shown in Figure 11A. The amount of Granzyme B production was increased in the GP130-IL6R-ca7R-transduced CD19-targeting CAR-T cells compared to the control cells (FMC63-28z CAR-T cells).

The quantification results of the amount of Granzyme B production in co-culture with different target cells are shown in Figure 11B. Both the K562-CD19 cell line and the Raji cell line showed that the amount of Granzyme B produced was increased in the GP130-IL6R-ca7R-transduced CD19-targeting CAR-T cells compared to the control cells.

### (3) Tumor cell survival rate

GP130-IL6R-ca7R-transduced CD19-targeting CAR-T cells and CD19-positive tumor cells (K562-CD19 cell line or Raji cell line) were co-cultured overnight at 1:1, and then the number of viable tumor cells was counted by flow cytometry. The viable tumor cells were identified by fluorescence derived from EGFP gene stably introduced into each cell line and by the LIVE/DEAD Fixable Near-IR Dead Cell Stain Kit (from Thermo Fisher Scientific, L10119), which selectively stained dead cells. The survival rate of the tumor cells in each condition was calculated with the number of viable tumor cells in the well in which only tumor cells were cultured as 100%.

The results of measuring tumor cell survival rate are shown in Figure 12. For both the K562-CD19 cell line or NALM6 cell line, the tumor cell survival rate was significantly lower in the GP130-IL6R-ca7R-transduced CD19-targeting CAR-T cells than the control cells, indicating that the GP130-IL6R-ca7R-transduced CD19-targeting CAR-T cells have an increased cytotoxic activity against the tumor cells.

### <Example 8: Effect of suppressive mutations Y449F, M452L, and Y456F>

### (Object)

In the intracellular domain of the IL-7 receptor α chain, Y449F, M452L, and Y456F, shown in Figure 13A, are known as mutations that suppress the activity of each functional motif. These suppressive mutations (loss-of-phosphorylation mutations) are introduced into the chimeric cytokine receptor GP130-IL6R-ca7R to examine their effects.

### (Methods and results)

### (1) Activity measurement of JAK-STAT pathway and Akt pathway

A CD19-targeting CAR gene and GP130-IL6R-ca7R gene were co-transduced into T cells. The GP130-IL6R-ca7R gene used included a GP130-IL6R-ca7R(WT) gene in which amino acid substitution mutation is not introduced, a GP130-IL6R-ca7R(Y449F) gene in which the amino acid substitution mutation Y449F is introduced, a GP130-IL6R-ca7R(M452L) gene in which the amino acid substitution mutation M452L is introduced, and a GP130-IL6R-ca7R(Y456F) gene in which the amino acid substitution mutation Y456F is introduced. After chimeric cytokine receptor-transduced CAR-T cells were allowed to rest in a cytokine-free medium for one day, intracellular proteins were collected and phosphorylated STAT3 (pSTAT3), phosphorylated STATS (pSTAT5), and phosphorylated Akt (pAkt) were detected by Western blot. At the same time, total STATS, total STATS, and total Akt, as well as β-actin as an internal control were detected.

The results are shown in Figure 13B. Activation of the JAK-STAT pathway and the Akt pathway was observed in the CAR-T cells co-transduced with GP130-IL6R-ca7R(WT), GP130-IL6R-ca7R(Y449F), or GP130-IL6R-ca7R(Y456F) was co-introduced, compared with the control cells. On the other hand, in the CAR-T cells transduced with GP130-IL6R-ca7R(M452L), the Akt pathway was not activated and only the JAK-STAT pathway was found to be selectively activated.

### (2) Evaluation of proliferation ability and immature phenotype of GP130-IL6R-ca7R (M452L)-transduced CAR-T cells.

The CAR gene targeting CD19 as an antigen and the GP130-IL6R-ca7R(WT) gene or GP130-IL6R-ca7R(M452L) gene were co-introduced into T cells. Each CAR-T cell was plated in a 48-well plate at 2.5 × 10⁵ cells/well and co-cultured at 1:1 with the CD19-positive tumor cell line NALM6. The cells were counted 7 days after the start of co-culture to evaluate their cell proliferation ability. The fold changes in the number of the cells from the start of co-culture are shown in Figure 14A. The CAR-T cells co-transduced with GP130-IL6R-ca7R(WT) and GP130-IL6R-ca7R(M452L) both showed significantly higher proliferation ability than the control cells.

The results of flow cytometry analysis for the cell surface expression of CD62L and CCR7 on each CAR-T cell after expansion are shown in Figure 14B. The CD62L and CCR7 are known to be highly expressed in memory T cells in an immature state (Gattinoni, L., et al., Nat Med., 2011, 17(10):1290-1297.). Compared to the control cells, the proportion of CD62L-positive CCR7-positive T cells corresponding to immature memory T cells decreased after expansion in the CAR-T cells co-transduced with GP130-IL6R-ca7R(WT), and the progression of differentiation was promoted (Figure 14B, 47.1% vs. 13.4%), whereas the progression of differentiation was suppressed in CAR-T cells co-transduced with GP130-IL6R-ca7R(M452L) compared to GP130-IL6R-ca7R(WT) (Figure 14B, 19.0% vs. 13.4%).

The fold change from the start of co-culture of the CAR-T cells having immature phenotypes are shown in Figure 14C. The proliferation rate of immature memory T cells was significantly increased in the CAR-T cells co-transduced with GP130-IL6R-ca7R(M452L) compared to both the control cells and the CAR-T cells co-transduced with GP130-IL6R-ca7R(M452L).

These results indicated that the introduction of the M452L mutation enables selective activation of only the JAK-STAT pathway without activation of the Akt pathway, thereby achieving maintenance of high proliferation ability and long-term survival ability while preventing excessive progression of T cell differentiation.

### <Example 9: Proliferation ability of chimeric cytokine receptor-transduced Mesothelin/GD2-targeting CAR-T cell>

### (Object)

The effect of the chimeric cytokine receptor introduction on the cell proliferation ability of CAR-T cells targeting mesothelin or GD2 as an antigen other than CD19 is evaluated.

### (Methods and results)

CAR genes targeting mesothelin (Mesothelin) or ganglioside GD2 and GP130-IL6R-ca7R genes were co-introduced into T cells. The CAR genes targeting mesothelin were used after genetic synthesis based on the amino acid sequence of the antibody clone ss1 with reference to the literature (Li, Q., et al., Anticancer Res., 2004, 24(3a):1327-35; Carpenito, C., et al., Proc Natl Acad Sci U S A., 2009, 106(9):3360-3365). The CAR genes targeting GD2 were used after gene synthesis based on the amino acid sequence in which an E101K mutation for increasing affinity was introduced into the antibody clone 14g2a with reference to the literature (Rossig, C., et al., Med Pediatr Oncol., 2000, 35 (6): 692-5; Horwacikm I., et al., Mol Cell Proteomics., 2015, 14 (10): 2577-90; Richman, S. A., et al., Cancer Immunol Res., 2018, 6 (1): 36-46.).

Hereinafter, T cells transduced with both the CAR gene targeting mesothelin or GD2 and the gene encoding the chimeric cytokine receptor (GP130-IL6R-ca7R gene) are referred to as "chimeric cytokine receptor-transduced mesothelin-targeting CAR-T cells" ("GP130-IL6R-ca7R-transduced mesothelin-targeting CAR-T cells") or "chimeric cytokine receptor-transduced GD2-targeting CAR-T cells" ("GP130-IL6R-ca7R-transduced GD2-targeting CAR-T cells")

The above CAR-T cells and control cells were cultured in the presence of IL-2, plated in 48-well plates at 2.5 × 10⁵ cells/well after removal of IL-2 on day 5 of culture, and co-cultured with cells expressing the antigen (mesothelin or GD2) targeted by each CAR. The GP130-IL6R-ca7R-transduced mesothelin-targeting CAR-T cells were co-cultured with mesothelin-stably transduced K562 cell line (referred to as a "K562-mesothelin cell line") at a ratio of 4:1. The K562-mesothelin cell line used was a K562 cell line (JCRB Cell Bank, JCRB0019) transduced with a mesothelin gene by retroviral vector, in which mesothelin-positive cells were isolated by flow cytometry. The GP130-IL6R-ca7R-transduced GD2-targeting CAR-T cells were co-cultured with GD2-stably transduced NALM6 cell line (referred to as a "NALM6-GD2 cell line") at a ratio of 1:1. The NALM6-GD2 cell line used was the above-mentioned NALM6 cell line into which a B4GALNT1 gene encoding GM2/GD2 synthase and a ST8SIA1 gene encoding GD3 synthase were co-transduced by retrovirus to obtain a clone that stably and highly expressed GD2 on the cell surface. The co-culture was performed under conditions without the addition of cytokines. The cell proliferation ability was evaluated by counting the cell number of the GP130-IL6R-ca7R-transduced mesothelin-targeting CAR-T cells and the GP130-IL6R-ca7R-transduced GD2-targeting CAR-T cells 7 days after the start of co-culture.

The fold change in the number of cells from the start of co-culture are shown in Figure 15. The GP130-IL6R-ca7R-transduced mesothelin-targeting CAR-T cells and the GP130-IL6R-ca7R-transduced GD2-targeting CAR-T cells showed significantly enhanced proliferation ability compared to the control cells.

### <Example 10: Cytokine production ability of chimeric cytokine receptor-transduced Mesothelin/GD2-targeting CAR-T cells>

### (Object)

The cytokine production ability of chimeric cytokine receptor-transduced mesothelin- or GD2-targeting CAR-T cells is evaluated.

### (Methods and results)

GP130-IL6R-ca7R-transduced mesothelin-targeting CAR-T cells were co-cultured with the K562-mesothelin cell line at 2:1. Brefeldin A was administered 2 hours after the start of co-culture. After another 4 hours, the T cells were stained with anti-IFN-y and anti-TNF-α antibodies. IFN-γ-producing cells, TNF-α-producing cells, and IFN-γ/TNF-α-producing cells in the CD8-positive T cell fraction were analyzed by flow cytometry.

The results are shown in Figure 16. In the GP130-IL6R-ca7R-transduced mesothelin-targeting CAR-T cells, the proportion of IFN-γ-producing cells (Figure 16B), TNF-α-producing cells (Figure 16C), and IFN-γ/TNF-α-producing cells (Figure 16D) were increased compared to the control cells.

Next, the GP130-IL6R-ca7R-transduced GD2-targeting CAR-T cells were co-cultured at 1:1 with the NALM6-GD2 cell line. The cytokine production ability in the CD8-positive T cells was evaluated in the same manner as described above.

The results are shown in Figure 17. In the GP130-IL6R-ca7R-transduced GD2-targeting CAR-T cells, the proportion of IFN-γ-producing cells (Figure 17A), TNF-α-producing cells (Figure 17B), and IFN-γ/TNF-α-producing cells (Figure 17C) were increased compared to the control cells.

### <Example 11: Production and functional analysis of chimeric cytokine receptor GP130-IL6R-CD28(T195P)>

### (Object)

A chimeric cytokine receptor GP130-IL6R-CD28(T195P) based on the CD28 receptor having a constitutively active mutation is produced and its function is evaluated.

### (Methods and results)

### (1) Production of chimeric cytokine receptor GP130-IL6R-CD28(T195P)

GP130-IL6R-CD28(T195P) was produced as an artificial receptor in which a signal peptide, a gp130-derived ligand-binding region, a linker peptide, an IL6RA-derived ligand-binding region, and a T-cell activating region were linked in this order from the N-terminal side (Figure 18B). Hereinafter, each component is described.

The signal peptide, the gp130-derived ligand-binding region, the linker peptide, and the IL6RA-derived ligand-binding region of GP130-IL6R-CD28(T195P) have the same configuration as GP130-IL6R-ca7R described above, and only the configuration of the T-cell activating region differs between them (Figure 2B, Figure 18B).

The T-cell activating region of GP130-IL6R-CD28(T195P) consists of a part from the hinge region to the intracellular domain of the CD28 receptor having a constitutively active mutation, the T195P mutation (known to increase the proliferation ability of the T cells; Yoo H.Y., et al. Nat Genet, 2014, 46(4) 371-5; Lee S. H., et al., Haematologica, 2015, 100(12): e505-e507). Specifically, the T-cell activating region consists of positions 114 to 220 of the constitutively active CD28 receptor having a substitution at position 195 from Thr residue to Pro residue in the full-length human CD28 receptor of SEQ ID NO: 35.

The full-length amino acid sequence of GP130-IL6R-CD28(T195P) is shown in SEQ ID NO: 36, and the base sequence of the gene encoding GP130-IL6R-CD28(T195P) (hereinafter referred to as a "GP130-IL6R-CD28(T195P) gene") is shown in SEQ ID NO: 18.

### (2) Functional analysis of chimeric cytokine receptor GP130-IL6R-CD28(T195P)

The above-described GP130-IL6R-CD28(T195P) gene, IL-6 receptor α chain (IL6RA) gene, IL6R-ca7R gene, or GP130-IL6R-ca7R gene was introduced into T cells together with the FMC63-28z CAR gene (hereinafter referred to as "GP130-IL6R-CD28(T195P)-transduced CD19-targeting CAR-T cells," "IL6R-transduced CD19-targeting CAR-T cells," "IL6R-ca7R-transduced CD19-targeting CAR-T cells," and "GP130-IL6R-ca7R-transduced CD19-targeting CAR-T cells," respectively). The cells were counted after co-culture with the CD19-positive tumor cell line NALM6 in the same manner as in Example 6 to evaluate their cell proliferation ability.

The fold change in the number of cells from the start of co-culture is shown in Figure 18C. No significant difference in cell proliferation ability was detected in the IL6R-transduced CD19-targeting CAR-T cells and the GP130-IL6R-CD28(T195P)-transduced CD19-targeting CAR-T cells compared to the control cells.

The results revealed that the chimeric cytokine receptor based on the constitutively active CD28 receptor did not have the effect of enhancing T-cell proliferation ability. As shown by the results, it is important that the T-cell activating region of the chimeric cytokine receptor was based on the constitutively active IL-7 receptor α chain for obtaining the advantageous effects of the present invention.

### <Example 12: Production and functional analysis of chimeric cytokine receptors CSF2RA-ca7R and CSF2RB-ca7R>

### (Object)

Chimeric cytokine receptors CSF2RA-ca7R and CSF2RB-ca7R, containing part of a Granulocyte macrophage colony-stimulating factor (GM-CSF) receptor α chain (hereinafter referred to as "CSF2RA") or β chain (hereinafter referred to as "CSF2RB") and part of the IL7RA are produced and their functions are evaluated.

### (Methods and results)

### (1) Production of chimeric cytokine receptors CSF2RA-ca7R and CSF2RB-ca7R

CSF2RA-ca7R and CSF2RB-ca7R were each produced as an artificial receptor in which a ligand-binding region and a T-cell activating region were linked in this order from the N-terminal side (Figures 19A and 19B). Hereinafter, each component is described.

The ligand-binding region of CSF2RA-ca7R consists of the signal peptide and the extracellular domain of the GM-CSF receptor α chain (CSF2RA), specifically positions 1 to 320 in the full-length human CSF2RA of SEQ ID NO: 20. The ligand-binding region of CSF2RB-ca7R consists of the signal peptide and the extracellular domain of the GM-CSF receptor β chain (CSF2RB), specifically positions 1 to 443 in the full-length human CSF2RB of SEQ ID NO: 22.

The T-cell activating regions of CSF2RA-ca7R and CSF2RB-ca7R consist of a part from the hinge region to the intracellular domain of IL7RA having a constitutively active mutation, more specifically, the amino acid sequence in which constitutively active insertion mutation Pro-Pro-Cys-Leu (SEQ ID NO: 2) is inserted between position 243 and position 244 in positions 232 to 459 of the full-length human IL7RA of SEQ ID NO: 1.

The full-length amino acid sequences of CSF2RA-ca7R and CSF2RB-ca7R are shown in SEQ ID NOs: 27 and 28, and the base sequences of a gene encoding CSF2RA-ca7R and CSF2RB-ca7R (hereinafter referred to as a "CSF2RA-ca7R gene" and a "CSF2RB-ca7R gene") are shown in SEQ ID NOs: 32 and 33.

### (2) Functional analysis of chimeric cytokine receptors CSF2RA-ca7R and CSF2RB-ca7R

Both the CSF2RA-ca7R and CSF2RB-ca7R genes described above were introduced into T cells together with the FMC63-28z CAR gene (hereinafter referred to as "CSF2RA-ca7R/CSF2RB-ca7R-transduced CAR-T cells"). The cells were counted after co-culture with the CD19-positive tumor cell line NALM6 in the same manner as in Example 6 to evaluate their cell proliferation ability. The fold change in the number of cells from the start of co-culture is shown in Figure 20A. A significant increase in cell proliferation was obtained as compared with the control cells into which only the CAR gene was introduced.

Next, the cells on day 5 of culture were plated at 2.5 × 10⁵ cells/well in a 48-well plate, and recombinant GM-CSF (PeproTech, Inc., 300-03) was added to a final concentration of 3,000 pg/mL. After 48 hours from the addition of GM-CSF, the culture supernatant was collected to measure GM-CSF concentration in the supernatant by ELISA. Note that in this Example, T cells transduced with only the NGFR-FMC63-28z CAR gene were used as control cells. The results of measuring the GM-CSF concentration in the culture supernatant are shown in Figure 20B. In the CSF2RA-ca7R/CSF2RB-ca7R-transduced CAR-T cells, the GM-CSF concentration in the supernatant decreased to below the detection limit compared to the control cells, indicating that the added GM-CSF was captured.

Similarly, the cells on day 5 of culture were plated at 2.5 × 10⁵ cells/well in a 48-well plate and co-cultured with 2.5 × 10⁵ cells of CD19-positive leukemic cell line NALM6 cells. After 24 hours, a monocytic leukemia cell line, THP1 cell line was added at 1.25 × 10⁵ cells/well. After another 24 hours, the culture supernatant was collected to measure the GM-CSF and IL-6 concentrations in the supernatant by ELISA. The results of measuring the GM-CSF concentration and the IL-6 concentration are shown in Figure 20C and Figure 20D, respectively. In the CSF2RA-ca7R/CSF2RB-ca7R-transduced CAR-T cells, the GM-CSF concentration in the supernatant was greatly reduced compared to the control cells, indicating that GM-CSF secreted from CAR-T cells themselves upon antigenic stimulation by NALM6 was captured. The results also showed that the monocytic cell line THP-1 secreted IL-6 upon GM-CSF secretion associated with CAR-T cell activation, while IL-6 secretion was suppressed upon capture of GM-CSF.

### <Example 13: Production and functional analysis of chimeric cytokine receptor IL1R2-ca7R>

### (Object)

A chimeric cytokine receptor IL1R2-ca7R containing part of the IL-1 receptor type 2 (hereinafter referred to as an "IL1R2") and part of IL7RA is produced and its function is evaluated.

### (Methods and results)

### (1) Production of Chimeric Cytokine Receptor IL1R2-ca7R

IL1R2-ca7R was produced as an artificial receptor in which a ligand-binding region and a T-cell activating region were linked in this order from the N-terminal side (Figures 19C). Hereinafter, each component is described.

The ligand-binding region consists of the signal peptide and extracellular domain of IL1R2, specifically, positions 1 to 343 in the full-length human IL1R2 of SEQ ID NO: 9.

The T-cell activating region consists of a part from the hinge region to the intracellular domain in IL7RA having a constitutively active mutation, specifically, the amino acid sequence in which constitutively active insertion mutation Pro-Pro-Cys-Leu (SEQ ID NO: 2) is inserted between position 243 and position 244 in positions 232 to 459 of the full-length human IL7RA of SEQ ID NO: 1.

The full-length amino acid sequence of IL1R2-ca7R is shown in SEQ ID NO: 26, and the base sequence of the gene encoding IL1R2-ca7R (hereinafter referred to as an "IL1R2-ca7R gene") is shown in SEQ ID NO: 31.

### (2) Functional analysis of chimeric cytokine receptor IL1R2-ca7R

The IL1R2-ca7R gene described above was introduced into T cells together with the FMC63-28z CAR gene (hereinafter referred to as "IL1R2-ca7R-transduced CD19-targeting CAR-T cells"). The cell number was counted after co-culture with the CD19-positive tumor cell line NALM6 in the same manner as in Example 6 to evaluate their cell proliferation ability.

The fold change in the number of cells from the start of co-culture is shown in Figure 21A. The proliferation rate of the IL1R2-ca7R-transduced CD19-targeting CAR-T cells was 10-fold or more, compared with less than 3-fold for control cells transduced with only the CAR gene.

Next, IL-1β capturing activity was analyzed in the same manner as in Example 4. Recombinant IL-1β (PeproTech, Inc., 200-01B) was added to the ILR2-ca7R-transduced CD19-targeting CAR-T cells to measure IL-1β concentration in the supernatant.

The results of measuring the IL-1β concentration in the culture supernatant are shown in Figure 21B. In the ILR2-ca7R-transduced CD19-targeting CAR-T cells, the IL-1β concentration in the supernatant was greatly reduced compared to the control cells transduced with only the CAR gene, and most of the added IL-1β was captured.

### <Example 14: In vivo IL-6 capture/absorption effect by chimeric cytokine receptor-transduced CAR-T cells>

### (Object)

Human IL-6 is administered via tail vein to NSG mice in which GP130-IL6R-ca7R-transduced CAR-T cells are administered and its effect on the change in blood IL-6 concentration is examined.

### (Methods and results)

An NALM6 cell line stably transduced with EGFP-P2A-Luc2 (GL) (referred to as a "NALM6-GL cell line") was administered at 2.5 × 10⁶ cells/mouse into the tail vein of the NSG mice (Charles River Laboratories Japan, JAX Mice Stock No: 005557). After 10 days, control cells transduced with only a CAR gene or GP130-IL6R-ca7R-transduced CAR-T cells were administered into the tail vein at 3 × 10⁶ cells/mouse. After another 7 days, 2 µg of human IL-6 was intravenously administered, and blood was collected at 1 hour, 2 hours, and 4 hours after the injection to measure the concentration of IL-6 in plasma by ELISA. Note that the control cells or GP130-IL6R-ca7R-transduced CAR-T cells used for the administration into the NSG mice were stimulated again with the NALM6-GL cell line on day 7 of culture and expanded, and cultured for 11 days.

The results are shown in Figure 22. The plasma IL-6 concentration was significantly lower in the NSG mice to which GP130-IL6R-ca7R-transduced CAR-T cells were administered compared to the NSG mice to which the control cells were administered, at all time points of 1 hour (Figure 22A), 2 hours (Figure 22B), and 4 hours (Figure 22C) after IL-6 administration. The results indicated that that IL-6 was captured/absorbed *in vivo* by the administration of the GP130-IL6R-ca7R-transduced CAR-T cells.

### <Example 15: Capture/absorption effect of IL-6 and IL-1β by CAR-T cell co-transduced with chimeric cytokine receptor and full-length IL-1 receptor type 2>

### (Object)

IL-6 or Il-1β which is recombinant or produced from a THP-1 cell line is added to CAR-T cells to which the chimeric cytokine receptor GP130-IL6R-ca7R(M452L) and the full-length IL-1 receptor type 2 are co-introduced, and the concentration of IL-6 or IL-1β in the supernatant is then measured to evaluate the ability of capturing IL-6 or IL-1β.

### (Methods and results)

### (1) Ability of capturing recombinant IL-6 and IL-1β

The GP130-IL6R-ca7R(M452L) gene was introduced into T cells together with a FMC63-28z-IL1R2 gene (hereinafter referred to as "GP130-IL-6R-ca7R(M452L) + FMC63-28z-IL1R2-transduced CAR-T cells"). FMC63-28z-IL1R2 is a polypeptide containing a FMC63-28z CAR, a porcine teschovirus-derived P2A sequence, and the full-length human IL-1 receptor type 2 consisting of the amino acid sequence of SEQ ID NO: 9. The above CAR-T cells on day 5 of culture were plated at 2.5 × 10⁵ cells/well in a 500 µL medium added with IL-2 (100 IU/mL) and IL-15 (10 ng/mL) in order to promote cell proliferation in a 48-well plate, and recombinant IL-6 or IL-1β was added to a final concentration of 3000 pg/mL. After 48 hours from the addition of IL-6, the culture supernatant was collected to measure IL-6 or IL-1β concentration in the supernatant by ELISA. The experimental procedure is shown in Figure 23-1A. Note that in this Example, T cells transduced with only the FMC63-28z CAR gene were used as control cells.

The results of measuring the IL-6 or IL-1β concentration in the culture supernatant are shown in Figure 23-1B and Figure 23-1C, respectively. In the GP130-IL-6R-ca7R (M452L) + FMC63-28z-IL1R2-transduced CAR-T cells, the IL-6 concentration in the supernatant was reduced below the detection limit (Figure 23-1B), and the IL-1β concentration was also greatly reduced compared to the control cells (Figure 23-1C).

### (2) Ability of capturing IL-6 and IL-1β produced by THP-1 cell line

The GP130-IL-6R-ca7R(M452L) + FMC63-28z-IL1R2-transduced CAR-T cells or control cells (T cells transduced with only the FMC63-28z CAR gene) on day 5 of culture were plated at 2.5 × 10⁵ cells/well in a 500 µL medium in a 48-well plate (without IL-2 or IL-15) and co-cultured with 2.5 × 10⁵ cells of the CD19-positive tumor cell line NALM6. After 24 hours, 1.25 × 10⁵ cells of the monocytic cell line THP-1 (JCRB Cell Bank, cell number: JCRB0112) were added. After 48 hours, the culture supernatant was collected to measure IL-6 or IL-1β concentration by ELISA. The experimental procedure is shown in Figure 23-2D.

The results of measuring the IL-6 or IL-1β concentration in the culture supernatant are shown in Figure 23-2E and Figure 23-2F, respectively. In the GP130-IL-6R-ca7R(M452L) + FMC63-28z-IL1R2-transduced CAR-T cells, the IL-6 concentration in the supernatant decreased to below the detection limit (Figure 23-2E), and the IL-1β concentration was also greatly reduced compared to the control cells, almost all of which were captured (Figure 23-2F).

These results indicated that both IL-6 and IL-1β could be captured with extremely high efficiency by CAR-T cells co-transduced with the chimeric cytokine receptor GP130-IL6R-ca7R(M452L) and the full-length IL-1 receptor type 2.

### <Example 16: Capture/absorption effect on IL-6 produced from monocytic cells in vivo>

### (Object)

GP130-IL6R-ca7R(M452L)-transduced CAR-T cells are administered to NSG mice to which the monocytic cell line THP-1 is administered via the tail vein and the blood concentration of IL-6 produced by the THP-1 cell line is evaluated.

### (Methods)

The monocytic cell line THP-1 stably transduced with CD19 and EGFP-Luc2 (hereinafter referred to as "THP1-CD19/EGFP-Luc2"), was administered into the tail vein of NSG mice at 3 × 10⁶ cells/mouse. After 24 to 29 days, the CAR-T cells co-transduced with the GP130-IL6R-ca7R(M452L) gene and the FMC63-28z gene (hereinafter referred to as "GP130-IL6R-ca7R(M452L)-transduced CAR-T cells") or control cells transduced with only the FMC63-28z gene were administered into the tail vein of mice at 5 × 10⁶ cells/mouse. After additional 1, 5, and 8 days, peripheral blood was collected to measure the plasma IL-6 concentration by ELISA (Figure 24A).

### (Results)

The results are shown in Figure 24B. NSG mice to which the GP130-IL6R-ca7R(M452L)-transduced CAR-T cells were administered showed significantly lower concentration of IL-6 in plasma at 1 and 5 days after administration of the THP1-CD19/EGFP-Luc2, compared to NSG mice to which the control cells were administered. The results indicated that IL-6 produced *in vivo* was captured/absorbed by the administration of the GP130-IL6R-ca7R(M452L)-transduced CAR-T cells.

### <Example 17: Evaluation of antitumor effect in in vivo leukemia model>

### (Object)

The GP130-IL6R-ca7R(M452L)-transduced CAR-T cells are administered into NSG mice to which the NALM6-GL cell line are administered via the tail vein to evaluate the antitumor effects in an *in vivo* leukemia model.

### (Methods)

The NALM6 cell line stably transduced with EGFP-P2A-Luc2 (GL) gene (NALM6-GL cell line) was administered at 2.5 × 10⁶ cells/mouse to NSG mice via the tail vein. After 3 days, the CAR-T cells co-transduced with the GP130-IL6R-ca7R(M452L) gene and the FMC63-28z gene (hereinafter referred to as "GP130-IL6R-ca7R(M452L)-transduced CAR-T cells") or control cells transduced with only the FMC63-28z gene were administered into the tail vein of mice at 0.5 × 10⁶ cells/mouse (Figure 25-1A). Note that the GP130-IL6R-ca7R(M452L)-transduced CAR-T cells or control cells administered to NSG mice were administered on day 9 of culture. Peripheral blood was collected at 10, 24, and 38 days after CAR-T cell administration to measure the proportion of human CD45-positive T cells by flow cytometry. In addition, NALM6-GL cells in the living body were detected by luciferase luminescence using an IVIS^{®} Spectrum *in vivo* imaging system (PerkinElmer, Inc.) at 0, 10, 24, 38, and 60 days after CAR-T cell administration. The progression-free survival rate of mice up to approximately 60 days was also analyzed with reference to the day of NALM6-GL cell transplantation. In the progression-free survival rate analysis, the endpoint was 20% or more weight loss.

### (Results)

The proportion of human CD45-positive T cells 10, 24, and 38 days after administration of the CAR-T cells are shown in Figure 25-1B. The proportion of human CD45-positive T cells were significantly higher in peripheral blood of NSG mice administered with the GP130-IL6R-ca7R(M452L)-transduced CAR-T cells than in those administered with the control cells 10 and 24 days after administration of the CAR-T cell, indicating the long-lasting properties of the GP130-IL6R-ca7R(M452L)-transduced CAR-T cells (Figure 25-1B).

The results of measuring the volume of NALM6 tumor as the amount of luciferase luminescence by IVIS Imaging are shown in Figure 25-2C and Figure 25-2D. The NSG mice administered with the GP130-IL6R-ca7R(M452L)-transduced CAR-T cells showed significantly lower volume of NALM6 tumor compared to those administered with control cells at all of 10, 24, and 38 days after administration of the CAR-T cell (Figure 25-2D). Furthermore, mice treated with the GP130-IL6R-ca7R(M452L)-transduced CAR-T cells showed significantly increased progression-free survival compared to mice administered with control cells (Figure 25-3E). These results revealed that the antitumor effect was enhanced in the GP130-IL6R-ca7R(M452L)-transduced CAR-T cells over a long period of time.

### <Example 18: Evaluation of antitumor effect in in vivo solid tumor model>

### (Object)

The GP130-IL6R-ca7R(M452L)-transduced CAR-T cells are administered into NSG mice subcutaneously implanted with mesothelin-positive pancreatic cancer cell line AsPC-1 as a solid tumor model to evaluate antitumor effects in an *in vivo* solid tumor model.

Note that in this Example, unlike Example 17, CAR-T cells, but not tumor cells, are labeled with luciferase.

### (Methods)

A mesothelin-positive pancreatic cancer cell line AsPC-1 (ATCC CRL-1682) was mixed at 1.5 × 10⁶ cells/mouse with Matrigel which is for promoting engraftment and then subcutaneously implanted into NSG mice. After 13 days from AsPC-1 transplantation, second-generation CAR-T cells co-transduced with the GP130-IL6R-ca7R(M452L) gene and a second-generation CAR, Luc2-ss1-28z gene (hereinafter referred to as "GP130-IL6R-ca7R(M452L)-transduced second-generation CAR-T cells") or control cells transduced with only the Luc2-ss1-28z gene were administered into the tail vein at 0.5 × 10⁶ cells/mouse (Figure 26-1A). Note that the GP130-IL6R-ca7R(M452L)-transduced second-generation CAR-T cells or control cells administered to NSG mice were administered on day 10 of culture. The Luc2-ss1-28z means Luc2-P2A-tNGFR-Furin-SGSG-P2A-ss1 28z, which is a polypeptide containing a luciferase gene, a P2A sequence, a truncated NGFR, a Furin protease cleavage sequence (RAKR), a flexible linker (SGSG), a P2A sequence, and an ss1 28z CAR in this order from the N-terminal side. Here, ss1 28z CAR is derived from the anti-mesothelin antibody clone ss1 and is a second-generation CAR gene having a CD28 signal domain for mesothelin. Luc2 contained in Luc2-ss1-28z allows CAR-T cell localization to be observed over time from outside the body based on luciferase luminescence. Peripheral blood was collected at 14, 21, 28, and 35 days after administration of the CAR-T cell to measure the proportion of human CD45-positive T cells by flow cytometry. CAR-T cells that were infiltrated into subcutaneous tumors were detected by luciferase luminescence using the IVIS^{®} Imaging System (PerkinElmer, Inc.) at 14, 21, 28, and 35 days after administration of the CAR-T cells. In addition, the progression-free survival rate of mice was analyzed up to approximately 80 days after the date of AsPC-1 cell transplantation. In the progression-free survival rate analysis, the endpoint was the first time point when the tumor volume was continuously 200 mm³ or more.

### (Results)

The proportion of human CD45-positive T cells at 14, 21, 28, and 38 days after administration of the CAR-T cell are shown in Figure 26-1B. The percentages of human CD45-positive T cells were significantly higher in peripheral blood of NSG mice administered with the GP130-IL6R-ca7R(M452L)-transduced second-generation CAR-T cells than in those administered with the control cells at 14, 21, and 28 days after administration of the CAR-T cell, indicating the long-lasting properties of the GP130-IL6R-ca7R(M452L)-transduced second-generation CAR-T cells (Figure 26-1B).

The results of measuring CAR-T cells that were infiltrated into subcutaneous tumors as the amount of luciferase luminescence by IVIS Imaging are shown in Figure 26-2C and Figure 26-2D. NSG mice administered with the GP130-IL6R-ca7R(M452L)-transduced second-generation CAR-T cells showed significantly enhanced CAR-T cell infiltration into the tumor (Figure 26-2D) and concomitantly significant reduction in tumor volume (Figure 26-3E) at all of 14, 21, and 28 days after administration of the CAR-T cell compared to those administered with control cells. Furthermore, mice administered with the GP130-IL6R-ca7R(M452L)-transduced second-generation CAR-T cells showed significantly increased progression-free survival period compared to mice administered with the control cells (Figure 26-3F). These results revealed that the antitumor effect on solid tumors was enhanced in the GP130-IL6R-ca7R(M452L)-transduced second-generation CAR-T cells over a long term.

All publications, patents, and patent applications cited herein are hereby incorporated by reference in their entirety.

## Claims

1. A chimeric cytokine receptor comprising a ligand-binding region at the N-terminal side and a T-cell activating region at the C-terminal side, wherein:
said ligand-binding region consists of a cytokine-binding region of a cytokine receptor,
said T-cell activating region comprises the transmembrane domain and the intracellular domain of an IL-7 (Interleukin-7) receptor α chain, and
said transmembrane domain has an insertion of any one selected from the group consisting of
(a) the amino acid sequence of SEQ ID NO: 2 between position 243 and position 244 in the amino acid sequence of SEQ ID NO: 1,
(b) the amino acid sequence of SEQ ID NO: 3 between position 241 and position 242 in the amino acid sequence of SEQ ID NO: 1,
(c) the amino acid sequence of SEQ ID NO: 4 between position 244 and position 245 in the amino acid sequence of SEQ ID NO: 1,
(d) the amino acid sequence of SEQ ID NO: 5 between position 244 and position 245 in the amino acid sequence of SEQ ID NO: 1, and
(e) the amino acid sequence of SEQ ID NO: 6 between position 246 and position 247 in the amino acid sequence of SEQ ID NO: 1.

2. The chimeric cytokine receptor of claim 1, wherein said cytokine receptor is selected from the group consisting of an IL-6 (Interleukin-6) receptor, an IL-1 (Interleukin-1) receptor type 2, a Granulocyte macrophage colony-stimulating factor (GM-CSF) receptor α chain, and a GM-CSF receptor β chain.

3. The chimeric cytokine receptor of claim 2, wherein said cytokine receptor is an IL-6 receptor and further comprises the ligand-binding region of gp130 (Glycoprotein 130) at the N-terminal side thereof.

4. The chimeric cytokine receptor of any one of claims 1 to 3, having a mutation which reduces the activity of one or more motifs selected from the group consisting of a JAK-binding motif, a STAT3 association motif, and a STAT5/PI3K association motif which are contained in said intracellular domain.

5. The chimeric cytokine receptor of claim 4, wherein said mutation is Y449F, M452L, or Y456F in the amino acid sequence of SEQ ID NO: 1.

6. A nucleic acid encoding the chimeric cytokine receptor of any one of claims 1 to 5.

7. A gene expression vector comprising the nucleic acid of claim 6 in a state which allows for the expression thereof.

8. A host cell comprising the gene expression vector of claim 7.

9. The host cell of claim 8, further comprising a chimeric antigen receptor (CAR) expression vector which comprises a base sequence encoding a CAR in a state which allows for the expression thereof.

10. The host cell of claim 8 or 9, further comprising an IL-1 receptor type 2 expression vector which comprises a base sequence encoding a full-length IL-1 receptor type 2 in a state which allows for the expression thereof.

11. The host cell of any one of claims 8 to 10 which is an immune cell.

12. The host cell of claim 11, wherein said immune cell is a T cell, an NK cell, or a macrophage.

13. A cell preparation comprising the host cell of any one of claims 8 to 12.

14. A method of producing a chimeric antigen receptor (CAR)-transduced cell having a long-lasting cytotoxic activity, comprising:
an isolating step of isolating peripheral blood mononuclear cells from the peripheral blood of a subject, and
an introducing step of introducing the gene expression vector of claim 7 and a CAR-expression vector to the peripheral blood mononuclear cells isolated in said isolating step,
wherein said CAR-expression vector comprises a base sequence encoding a chimeric antigen receptor (CAR) in a state which allows for the expression thereof.
